(19) Europäisches Patentamt
European Patent Office
Office européen des brevets

(11) Veröffentlichungsnummer: **0 222 962**
**B1**

(12) # EUROPÄISCHE PATENTSCHRIFT

(45) Veröffentlichungstag der Patentschrift: **24.01.90**

(51) Int. Cl.[4]: **C07D 257/04**, A61K 31/41

(21) Anmeldenummer: **85810551.3**

(22) Anmeldetag: **18.11.85**

(54) **Weitere neue Resorcinäther.**

(43) Veröffentlichungstag der Anmeldung: **27.05.87 Patentblatt 87/22**

(45) Bekanntmachung des Hinweises auf die Patenterteilung: **24.01.90 Patentblatt 90/4**

(84) Benannte Vertragsstaaten: **AT BE CH DE FR GB IT LI LU NL SE**

(56) Entgegenhaltungen:
**EP-A- 0 123 541**
**EP-A- 0 132 366**
**EP-A- 0 132 367**
**EP-A- 0 147 973**
**DE-A- 2 846 931**
**US-A- 4 448 729**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Beck, Andreas, Dr., Kohlerweg 23, D-7800 Freiburg(DE)**
Erfinder: **Sallmann, Alfred, Dr., Joachimsackerstrasse 12, CH-4103 Bottmingen(CH)**
Erfinder: **Wenk, Paul, Dr., Quellenweg 7, CH-4123 Allschwil(CH)**

**EP 0 222 962 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 4-Acylresorcinäther der Formel

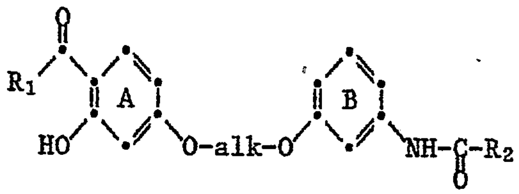

(I),

worin $R_1$ Niederalkyl bedeutet, alk einen gegebenenfalls durch Sauerstoff unterbrochenen Hydroxyniederalkylenrest darstellt und $R_2$ 5-Tetrazolyl bedeutet, wobei der Ring A zusätzlich durch Niederalkyl, Niederalkenyl, Niederalkinyl, Niederalkoxy und/oder Halogen und der Ring B zusätzlich durch Niederalkyl, Niederalkanoyl, Niederalkoxy, Niederalkoxycarbonyl, Carboxy, Carbamyl, Cyano, Halogen und/oder Trifluormethyl substituiert sein kann und wobei Halogen eine Atomnummer bis und mit 53 aufweisen kann und "niedere" organische Gruppen bis und mit 7 C-Atome besitzen können, und ihre Salze.

Gegebenenfalls durch Sauerstoff unterbrochene Hydroxyniederalkylenreste alk sind beispielsweise, vorzugsweise geradkettige, Hydroxyniederalkylenreste bzw. Hydroxy(oxa)niederalkylen- bzw. Hydroxy(dioxa)niederalkylenreste, worin die Hydroxygruppe in höherer als der α- und in niedrigerer als der ω-Stellung zu den freien Valenzen sowie in höherer als der α-Stellung zu (m) gegebenenfalls vorhandenen, in höherer als der β- und in niedrigerer als der (ω-1)-Stellung zu den freien Valenzen befindlichen, Oxaglied(ern) gebunden ist. Solche Reste sind insbesondere Reste der Formel

$$-[-(CH_2)_n-O-]-CH_2-CH(OH)-CH_2-[-O-(CH_2)_{n'}-]_p- \qquad (Ib)$$

worin n und n' unabhängig voneinander 2, 3 oder 4 bedeuten und o sowie p unabhängig voneinander für 0 oder 1 stehen, wobei n, n' und p jedoch nur solche Werte annehmen können, daß die Reste Ib insgesamt 3 bis und mit 7 C-Atome aufweisen, vor allem Hydroxyniederalkylen- bzw. Hydroxy(oxa)niederalkylenreste der Formel Ib, worin die Indizes o und p 0 bedeuten bzw. einer der Indizes o und p für 1 und der andere für 0 und n bzw. n' für 2 steht.

Vor- und nachstehend sind unter "niederen" organischen Verbindungen und davon abgeleiteten Gruppen beispielsweise solche zu verstehen, die bis und mit 7, insbesondere bis und mit 4, Kohlenstoffatome (C-Atome) aufweisen.

Niederalkyl bedeutet beispielsweise Methyl, Aethyl, Propyl, Isopropyl oder Butyl, ferner Sekundär- oder Tertiärbutyl, im Falle von $R_1$ insbesondere Methyl und im Falle eines Niederalkylsubstituenten des Ringes A insbesondere Propyl.

Niederalkoxy bedeutet beispielsweise Methoxy, Aethoxy, Propyloxy, Isopropyloxy oder Butyloxy.

Halogen weist beispielsweise die Atomnummer bis und mit 53, insbesondere 17 bis und mit 53, auf und bedeutet beispielsweise Fluor, Chlor, Brom oder Iod.

Niederalkenyl ist beispielsweise Allyl, ferner Methallyl oder But-4-enyl.

Niederalkinyl ist beispielsweise Propargyl.

Niederalkanoyl ist beispielsweise Formyl, Acetyl, Propionyl, Butyryl, Valeroyl oder Pivaloyl.

Hydroxyniederalkylen, worin die Hydroxygruppe in höherer als der α- und in niedrigerer als der ω-Stellung zu den freien Valenzen gebunden ist, bedeutet beispielsweise 1,3-(2-Hydroxy)propylen, kann aber auch 1,4-(2-Hydroxy)butylen oder 1,5-(3-Hydroxy)pentylen sein.

Hydroxy(oxa)niederalkylen, worin die Hydroxygruppe in höherer als der α- und in niedrigerer als der ω-Stellung zu den freien Valenzen sowie in höherer als der α-Stellung zum Oxaglied, welches sich seinerseits in höherer als der β- und in niedrigerer als der (ω-1)-Stellung zu den freien Valenzen befindet, gebunden ist, bedeutet beispielsweise 1,7-(2-Hydroxy-4-oxa)heptylen, kann aber auch 1,6-(2-Hydroxy-4-oxa)hexylen sein.

Niederalkoxycarbonyl ist beispielsweise Methoxy-, Aethoxy-, Propyloxy-, Isopropyloxy- oder Butyloxycarbonyl.

Als Salze von Verbindungen der Formel I kommen vorzugsweise pharmazeutisch verwendbare Salze in Frage, wie Metallsalze, Ammoniumsalze oder Salze mit organischen Basen. Metallsalze sind beispielsweise entsprechende Alkali- oder Erdalkalimetallsalze, z.B. Lithium-, Natrium-, Kalium-, Magnesium- oder Calciumsalze, ferner pharmazeutisch verwendbare Übergangsmetall-, wie Zink- oder Kupfersalze. Salze mit organischen Basen werden beispielsweise von Verbindungen der Formel I mit mono-, di- oder trisubstituierten organischen Aminen, wie entsprechenden Alkylaminen, Hydroxyalkylaminen, geeigneten, mindestens ein N-Atom aufweisenden Heterocyclen, wie Morpholin, Thiomorpholin, Piperidin oder Pyrrolidin, gegebenenfalls N-substituierten Amino-sacchariden, z.B. mit N-Methyl-D-glucamin, oder basischen Aminosäuren, wie Lysin, Arginin, Histidin oder Ornithin, wobei jene mit L-Konfiguration bevorzugt sind, gebildet. Als Alkylamine kommen z.B. Mono-, Di- oder Triniederalkylamine, wie Aethyl-, tert-Butyl-, Diäthyl-, Diisopropyl-, Trimethyl- oder Triäthylamin, in Betracht. Hydroxyalkylamine sind beispielsweise Mono-, Di- oder Trihydroxyalkylamine, wie Mono-, Di- oder Triäthanolamin oder Diisopropanolamin, oder Hydroxyniederalkyl-niederalkylamine, wie N,N-Dimethyl- oder N,N-Diäthylaminoäthanol oder Tris(hydroxymethyl)methylamin.

Als weitere Salze sind pharmazeutisch verwendbare Säureadditionssalze, wie Hydrohalogenide, Methansulfonate, N-Cyclohexylsulfaminate, Maleinate, Maleate oder Fumarate, von Verbindungen der Formel I zu nennen.

Die Verbindungen der Formel I mit chiralen C-Atomen können je nach der Anzahl derselben in zueinander enantiomeren bzw. diastereomeren Formen oder als Gemische derselben, wie Diastereomerengemische, Racemate oder Racematgemische, vorliegen.

Die erfindungsgemäßen Verbindungen zeichnen sich durch wertvolle pharmakologische Eigenschaften aus. Insbesondere besitzen sie eine ausgeprägte antiallergische Aktivität, die sich aufgrund eines deutlichen $LTD_4$-Antagonismus sowie eines PAF-Antagonismus (PAF-acether antagonism, in vitro) ableiten läßt, da die erfindungsgemäßen Verbindungen in vitro im Bereich von etwa 0,005 bis etwa 0,05 µmol/l durch $LTD_4$ induzierte Kontraktionen hemmen. Der $LTD_4$-Antagonismus kann z.B. am isolierten Meerschweinchen-Ileum nachgewiesen werden. Hierzu werden an Ileumsegmenten, die aus Meerschweinchen von 300 bis 400 g Körpergewicht entnommen, in einem Organbad in Tyrodelösung (38°C, Begasung mit einem Gemisch aus 95% $O_2$ und 5% $CO_2$) befestigt und mit lp belastet wurden, durch synthetisches $LTD_4$ (Leukotrien $D_4$, Kaliumsalz) Kontraktionen ausgelöst, deren Ausmaß registriert wird. Das Ausmaß der auf die $LTD_4$-antagonistische Wirkung der Testsubstanz zurückführende Hemmung dieser Kontraktionen wird gemessen. Man ermittelt diejenige, als $IC_{50}$ bezeichnete Konzentration der Testsubstanz, die durch $LTD_4$ induzierte Kontraktionen auf 50% des Ausgangswertes reduziert. In dieser Versuchsanordnung wurden beispielsweise für das erfindungsgemäße Triäthanolammoniumsalz von N-{3-[3-(4-Acetyl-3-hydroxy-2-propyl-phenoxy)-2-hydroxy-propyloxy]-4-brom-6-methylphenyl}-1H-tetrazol-5-carboxamid ein $IC_{50}$-Wert von 0,0054 µmol/l bzw. für das erfindungsgemäße Triäthylammoniumsalz von N-{3-[3-(4-Acetyl-3-hydroxy-2-propyl-phenoxy)-2-hydroxy-propyloxy]-2-cyano-phenyl}-1H-tetrazol-5-carboxamid ein $IC_{50}$-Wert von 0,03 µmol/l und für N-{3-[3-(4-Acetyl-3-hydroxy-2-propyl-phenoxy)-2-hydroxy-propyloxy]-phenyl}-oxaminsäureäthylester bzw. für das Natriumsalz von N-{3-[3-(4-Acetyl-3-hydroxy-2-propyl-phenoxy)-2-hydroxy-propyloxy]-2-cyano-phenyl}-oxaminsäure, beide gemäß US-Patentschrift Nr. 4 448 729, ein $IC_{50}$-Wert von 0,087 µmol/l erhalten.

Die $LTD_4$-antagonistische Wirkung der neuen Verbindungen kann auch in vivo anhand der Hemmung experimenteller $LTD_4$-Bronchospasmen des Meerschweinchens nachgewiesen werden.

In der erwähnten US-Patentschrift werden unter anderem schon Verbindungen mit antiallergischer Wirksamkeit offenbart, die von der Grundstruktur her den Verbindungen der Formel I ähneln, jedoch anstelle der 5-Tetrazolylgruppe $R_2$ einen Rest der Formel –COOZ aufweisen, worin Z Wasserstoff, $C_1$–$C_6$-Alkyl oder ein Äquivalent eines pharmazeutisch verwendbaren Kations bedeutet. In der europäischen Patentanmeldung Nr. 0 147 973 werden zudem unter anderem bereits Verbindungen mit antiallergischer und antiinflammatorischer Wirksamkeit sowie Leukotriensynthese-hemmenden Eigenschaften beschrieben, die ebenfalls eine ähnliche Grundstruktur wie die Verbindungen der Formel I besitzen, sich von diesen jedoch unter anderem dadurch unterscheiden, daß sie anstelle der gegebenenfalls durch Sauerstoff unterbrochenen Hydroxyniederalkylengruppe alk eine unsubstituierte Alkylengruppe der Formel $-(CH_2)_n-$ aufweisen, wobei n 2, 3, 4 oder 5 bedeutet.

Die Erfindung betrifft in erster Linie Erfindungen der Formel I, worin $R_1$ Niederalkyl bedeutet, alk für Hydroxyniederalkylen, Hydroxy(oxa)niederalkylen oder Hydroxy(dioxa)niederalkylen, worin die Hydroxygruppe in höherer als der α- und in niedrigerer als der ω-Stellung zu den freien Valenzen sowie in höherer als der α-Stellung zu(m) gegebenenfalls vorhandenen, in höherer als der β- und in niedrigerer als der (ω-1)-Stellung zu den freien Valenzen befindlichen, Oxaglied(ern) gebunden ist, steht und $R_2$ 5-Tetrazolyl bedeutet, wobei der Ring A zusätzlich durch Niederalkyl, Niederalkenyl, Niederalkinyl, Niederalkoxy und/oder Halogen und der Ring B zusätzlich durch Niederalkyl, Niederalkanoyl, Niederalkoxy, Niederalkoxycarbonyl, Carbamyl, Carboxy, Cyano, Halogen und/oder Trifluormethyl substituiert sein kann und wobei Halogen eine Atomnummer bis und mit 53 aufweisen kann und "niedere" organische Gruppen bis und mit 7 C-Atome besitzen können, und ihre Salze, insbesondere pharmazeutisch verwendbaren Salze.

Die Erfindung betrifft vor allem Verbindungen der Formel

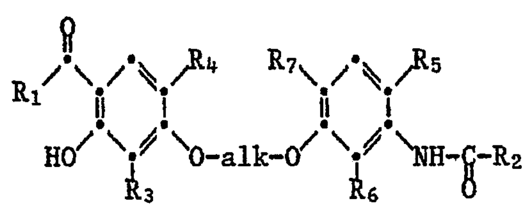

(Ia) ,

worin $R_1$ für Niederalkyl mit bis und mit 4 C-Atomen, wie Methyl, steht, $R_2$ 5-Tetrazolyl bedeutet, $R_3$ Niederalkyl mit bis und mit 4 C-Atomen, wie Propyl, darstellt, $R_4$ für Wasserstoff, Niederalkyl mit bis und mit 4 C-Atomen, wie Methyl, Niederalkoxy mit bis und mit 4 C-Atomen, wie Methoxy, oder Halogen der Atomnummer bis und mit 53, wie Chlor oder Brom, steht, $R_5$ für Wasserstoff, Niederalkyl mit bis und mit 4 C-Atomen, wie Methyl, Halogen der Atomnummer bis und mit 53, wie Chlor oder Brom, oder Trifluormethyl steht, $R_6$ Wasserstoff, Niederalkyl mit bis und mit 4 C-Atomen, wie Methyl, Niederalkoxy mit bis und mit 4 C-Atomen, wie Methoxy, Cyano oder Halogen der Atomnummer bis und mit 53, wie Chlor oder Brom, darstellt, $R_7$ Wasserstoff, Niederalkyl mit bis und mit 4 C-Atomen, wie Methyl, Halogen der Atomnummer bis und mit 53, wie Chlor oder Brom, Niederalkoxycarbonyl mit bis und mit 5 C-Atomen, wie Aethoxycarbonyl, Carboxy, Cyano oder Niederalkanoyl mit bis und mit 7 C-Atomen, wie Formyl, Acetyl oder Pivaloyl, bedeutet und alk für 1,3-(2-Hydroxy)propylen steht, und ihre Salze, insbesondere pharmazeutisch verwendbaren Salze.

Die Erfindung betrifft in allererster Linie Verbindungen der Formel Ia, worin $R_1$ Niederalkyl mit bis und mit 4 C-Atomen, wie Methyl, bedeutet, $R_3$ geradkettiges Niederalkyl mit bis und mit 4 C-Atomen, wie Propyl, bedeutet, $R_2$ 5-Tetrazolyl darstellt, $R_4$ für Wasserstoff steht, $R_5$ Niederalkyl mit bis und mit 4 C-Atomen, wie Methyl, bedeutet, $R_6$ Wasserstoff darstellt, $R_7$ für Halogen mit einer Atomnummer von 17 bis und mit 53, wie Chlor oder Brom, oder Cyano steht und alk 1,3-(2-Hydroxy)propylen bedeutet, und ihre Salze, insbesondere pharmazeutisch verwendbaren Salze, mit Basen.

Die Erfindung betrifft namentlich die in den Beispielen genannten Verbindungen der Formel I und ihre Salze, insbesondere pharmazeutisch verwendbaren Salze, mit Basen.

Die Erfindung betrifft ferner ein auf an sich bekannten Methoden beruhendes Verfahren zur Herstellung von Verbindungen der Formel I oder ihrer Salze. Dieses ist dadurch gekennzeichnet, daß man

a) eine Verbindung der Formel

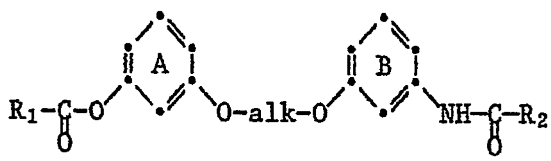

(II)

umlagert oder

b) eine Verbindung der Formel

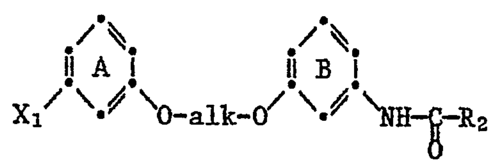

(III),

worin $X_1$ gegebenenfalls veräthertes Hydroxy bedeutet, mit einer Verbindung der Formel $R_1–X_2$ (IV), worin $X_2$ gegebenenfalls funktionell abgewandeltes Carboxy bedeutet, umsetzt oder

c) in einer Verbindung der Formel

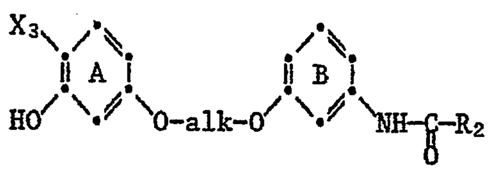

(V) ,

worin $X_3$ einen in die Gruppe der Formel $R_1–C(=O)–$ überführbaren Rest bedeutet, $X_3$ in diese Gruppe überführt oder

d) in einer Verbindung der Formel

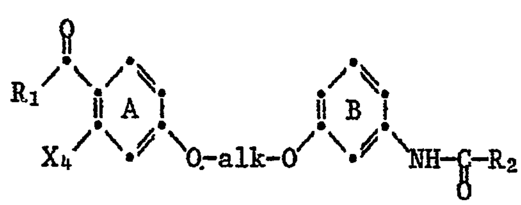

(VI),

worin $X_4$ einen in Hydroxy überführbaren Rest bedeutet, $X_4$ in Hydroxy überführt oder

e) Verbindungen der Formeln

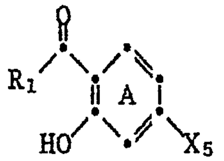 (VII) und 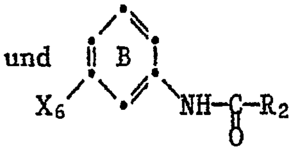 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest darstellt, wobei "niedere" organische Gruppen 3 bis und mit 7 C-Atome besitzen können, oder

f) in einer Verbindung der Formel

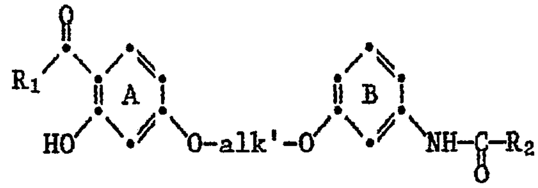 (IX),

worin alk' eine in einen Rest alk überführbare Gruppe bedeutet, alk' in einen Rest alk überführt oder

g) eine Verbindung der Formel

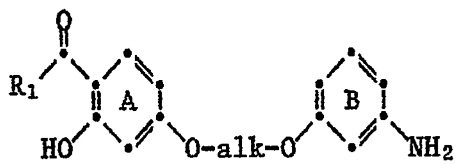 (X)

oder ein Salz davon mit einer Verbindung der Formel

$X_7$–$R_2$ (XI)

umsetzt, worin $X_7$ eine gegebenenfalls veresterte, amidierte, anhydridisierte oder in Salzform vorliegende Carboxygruppe bedeutet, oder

h) in einer Verbindung der Formel

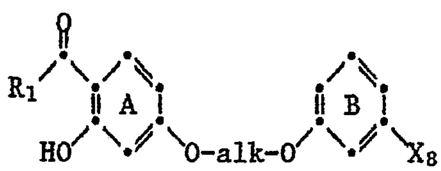 (XII),

worin $X_8$ einen in die gewünschte Gruppe der Formel –NH–C(=O)–$R_2$ überführbaren Rest bedeutet, $X_8$ in diese überführt und gewünschtenfalls eine verfahrensgemäß erhältliche Verbindung der Formel I in eine andere Verbindung der Formel I überführt und/oder eine verfahrensgemäß erhältliche freie Verbindung der Formel I in ein Salz oder ein verfahrensgemäß erhältliches Salz in die freie Verbindung der Formel I oder in ein anderes Salz umwandelt.

Gegebenenfalls veräthertes Hydroxy $X_1$ in Formel III bedeutet beispielsweise freies oder aliphatisch veräthertes Hydroxy, wie Hydroxy oder Niederalkoxy, z.B. Methoxy, kann aber auch araliphatisch, cycloaliphatisch oder aromatisch veräthertes Hydroxy, wie gegebenenfalls substituiertes Phenylalkoxy, z.B. Benzyloxy, Cycloalkoxy, z.B. Cyclohexyloxy oder Cyclopentyloxy, oder gegebenenfalls substituiertes Phenoxy, sein.

Gegebenenfalls funktionell abgewandeltes Carboxy $X_2$ in Formel IV bedeutet beispielsweise freies, verestertes oder anhydridisiertes Carboxy, wie Carboxy, Niederalkoxycarbonyl, Halogencarbonyl oder anhydridisiertes Carboxy der Formel –C(=O)–O–C(=O)–$R_1$.

In die Gruppe $R_1$–C(=O)– überführbare Reste $X_3$ in Formel V sind beispielsweise Gruppen der Formel $R_1$–CH($X_9$)–, worin $X_9$ Wasserstoff oder gegebenenfalls mit einer organischen Carbonsäure verestertes Hydroxy ist, oder Gruppen der Formel $R_1$–C(=NH)–, ferner gegebenenfalls funktionell abgewandelte Carboxygruppen, wie freies, in Salzform vorliegendes oder verestertes Carboxy oder Cyano. Mit einer

organischen Carbonsäure verestertes Hydroxy ist beispielsweise Niederalkanoyloxy, wie Acetoxy, kann aber auch gegebenenfalls substituiertes Benzyloxy sein. Als Salzformen von Carboxy $X_3$ kommen beispielsweise Alkalimetall-, Erdalkalimetall- oder Ammoniumsalzformen in Betracht, z.B. Natrium- oder Ammoniumsalzformen oder Halogenmagnesiumsalzformen. Verestertes Carboxy $X_3$ ist beispielsweise Niederalkoxycarbonyl, kann aber auch gegebenenfalls substituiertes Phenoxycarbonyl bzw. Phenylniederalkoxycarbonyl, wie Benzyloxycarbonyl, sein.

In Hydroxy überführbare Reste $X_4$ in Formel VI sind beispielsweise verätherte oder veresterte Hydroxygruppen. Als veräthertes Hydroxy $X_4$ kommen beispielsweise aliphatisch veräthertes Hydroxy, z.B. Niederalkoxy, wie Methoxy, oder Niederalkenyloxy, insbesondere Niederalk-2-enyloxy, z.B. Allyloxy, Phenylniederalkoxy, insbesondere gegebenenfalls substituiertes Phenylniederalkoxy, wie Benzyloxy, ferner Tetrahydropyran-2-yloxy oder Silyloxy, insbesondere Triniederalkylsilyloxy, z.B. Trimethylsilyloxy, in Betracht. Verestertes Hydroxy $X_4$ ist beispielsweise mit einer Carbonsäure, wie einer aliphatischen oder aromatischen Carbonsäure, oder mit einem aliphatischen oder aromatischen Halbester der Kohlensäure verestertes Hydroxy, wie Niederalkanoyloxy, z.B. Acetoxy, gegebenenfalls substituiertes Benzyloxy, z.B. der Formel $R_1-C(=O)-O-$, gegebenenfalls halogeniertes Niederalkoxycarbonyloxy, z.B. Mehoxy-, Aethoxy- oder Tertiärbutyloxycarbonyloxy, 2,2,2-Triiodäthoxy- oder 2,2,2-Trichloräthoxycarbonyloxy, gegebenenfalls substituiertes Phenylniederalkoxycarbonyloxy, insbesondere 1-Phenylniederalkoxycarbonyloxy, z.B. Benzyloxycarbonyloxy, oder gegebenenfalls substituiertes Phenoxycarbonyloxy.

In Salzform vorliegendes Hydroxy $X_5$ in Formel VII bzw. $X_6$ in Formel VIII bzw. Carboxy $X_7$ in Formel XI liegt insbesondere in einer Alkalimetallsalzform, z.B. als Natrium- oder Kaliumsalz, vor.

Gegebenenfalls durch Sauerstoff unterbrochene, durch reaktionsfähige veresterte Hydroxygruppen substituierte Hydroxyniederalkoxyreste bzw. ggf. durch Sauerstoff unterbrochene Epoxyniederalkoxyreste $X_5$ in Formel VII bzw. $X_6$ in Formel VIII sind beispielsweise Epoxyniederalkoxy- bzw. Epoxy-(oxa)niederalkoxyreste, insbesondere der Formel

$$-O-[-(CH_2)_n-O-]_o-CH_2-X' \text{ bzw. } X'-CH_2-[-O-(CH_2)_{n'}-]_p-O-,$$

reaktionsfähige monoveresterte Dihydroxyniederalkoxyreste, in denen die freie Hydroxygruppe in höherer als der α-Stellung zu dem die freie Valenz tragenden Sauerstoffatom und zur reaktionsfähigen veresterten Hydroxygruppe gebunden ist, oder entsprechende reaktionsfähige monoveresterte Dihydroxy(mono- oder dioxa)niederalkoxyreste, insbesondere der Formel

$$-O-[-(CH_2)-O-]-CH_2-CH(OH)-CH_2-[-O-(CH_2)_{n'}-]_p-X \text{ bzw.}$$
$$X-[-(CH_2)_n-O-]_o-CH_2-CH(OH)-CH_2-[-O-(CH_2)_{n'}-]_p-O-,$$

worin X jeweils reaktionsfähiges verestertes Hydroxy, X' 1,2-Epoxyäthyl, n und n' 2, 3 oder 4, o 0 oder 1 und p 0 oder 1 bedeuten, wobei für die möglichen Werte von n, n', o und p jedoch die vorstehend bei den Resten alk angegebene Bedingung in entsprechender Weise gilt. Reaktionsfähiges verestertes Hydroxy ist dabei beispielsweise Halogen, wie Chlor, Brom oder Iod, oder organisches Sulfonyloxy, wie Niederalkansulfonyloxy, z.B. Methansulfonyloxy, oder gegebenenfalls substituiertes Benzolsulfonyloxy, z.B. Benzol-, p-Brombenzol- oder p-Toluolsulfonyloxy. Vorzugsweise steht n bzw. n' für 2, o bzw. p für 0 und p bzw. o für 0 oder in zweiter Linie für 1.

In Reste –alk– überführbare Reste –alk'– in Formel IX sind beispielsweise entsprechende Oxoniederalkylenreste bzw. Oxo(mono- oder dioxa)niederalkylenreste, insbesondere der Formeln

$$-[-(CH_2)_n-O-]_o-(CH_2)-C(=O)-CH_2-[-O-(CH_2)_{n'}]_p-,$$

oder verätherte bzw. veresterte Hydroxyniederalkylen- bzw. Hydroxy(mono- oder dioxa)niederalkylenreste, insbesondere der Formel

$$-[-(CH_2)_n-O-]_o-CH_2-CH(X'')-CH_2-[-O-(CH_2)_{n'}-]_p-,$$

worin X" veräthertes oder verestertes Hydroxy, n und n' 2, 3 oder 4, o 0 oder 1 und p 0 oder 1 bedeuten, wobei für die möglichen Werte von n, n', o und p jedoch die vorstehend bei den Resten alk angegebene Bedingung in entsprechender Weise gilt.

Veräthertes Hydroxy X" ist dabei beispielsweise mit eine α-Aralkanol oder einem Silanol veräthertes Hydroxy, wie gegebenenfalls substituiertes Benzyloxy oder Triniederalkylsilyloxy, z.B. Trimethylsilyloxy.

Verestertes Hydroxy X" ist beispielsweise mit einer Carbonsäure, wie einer Niederalkansäure, oder einem Halbester der Kohlensäure verestertes Hydroxy, wie Niederalkanoyloxy, z.B. Acetoxy oder Pivaloyloxy, Niederalkoxycarbonyloxy, z.B. Tertiärbutoxycarbonyloxy, oder gegebenenfalls substituiertes Benzyloxycarbonyloxy, z.B. Benzyloxycarbonyloxy.

Gegebenenfalls verestertes, amidiertes oder anhydridisiertes Carboxy $X_7$ in Formel XI ist beispielsweise freies Carboxy, verestertes Carboxy, mit einem gegebenenfalls substituierten Phenol verestertes Carboxy, wie Phenoxy-, 4-Nitrophenoxy- oder 2,4-Dinitrophenoxycarbonyl, amidiertes Carboxy, aktiviertes Carbamyl, wie 1-Imidazolyl- bzw. 1-(2,5-Dimethyl-imidazolyl)carbonyl, oder mit einer Halogenwasserstoffsäure anhydridisiertes Carboxy, wie Halogencarbonyl, z.B. der Formel Hal–C(=O)–, worin Hal Chlor, Brom oder Iod, vor allem Chlor, ist.

Als Ausgangsstoffe XI kommen insbesondere in Metallsalzform, z.B. in Kaliumsalzform, vorliegende Carboxygruppen oder Reste der Formel $-Hal-C(=O)-R_2$ (XIb) in Betracht. In geschützter Form vorliegende 5-Tetrazolylreste sind beispielsweise gegebenenfalls im Arylteil substituierte 1-(α-Aralkyl)-tetrazolyl-(5)-reste, wie 1-Benzyltetrazolyl-(5) oder 1-(p-Methoxybenzyl)-tetrazolyl-(5).

Ein in die Gruppe der Formel $-NH-C(=O)-R_2$ überführbarer Rest $X_8$ in Formel XII ist beispielsweise die Gruppe der Formel $-NH-C(=O)-CN$.

Die Durchführung der verfahrensgemäßen Reaktionen sowie die Herstellung der neuen Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, Reaktions-, wie Temperatur- und Druckbedingungen, sowie gegebenenfalls Schutzgase verwendet.

Die Umlagerung von Verbindungen II gemäß Verfahrensvariante a) erfolgt beispielsweise photochemisch oder in Gegenwart eines sauren Kondensationsmittels, vorzugsweise in einem inerten Lösungsmittel. Geeignete saure Kondensationsmittel sind beispielsweise Lewissäuren, insbesondere komplexe Metallhalogenide der Formel $M^nY_n$ (XIX), worin M ein n-wertiges, koordinativ ungesättigtes Metallatom der Gruppe IIb, IIIa, IIIb, IVb, Va oder VIIIb des periodischen Systems der Elemente, z.B. ein $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$-atom, und Y ein Halogenatom, insbesondere der Atomnummer bis und mit 35, wie Fluor, Chlor oder Brom, darstellt. Vorzugsweise verwendet man Bortrifluorid, Aluminiumtrichlorid, Galliumchlorid, Zinntetrachlorid oder Zinkchlorid. Weitere geeignete saure Kondensationsmittel sind komplexe Sauerstoffsäuren, vor allem des Schwefels oder Phosphors, wie Schwefelsäure, Pyroschwefelsäure, Phosphorsäure, Pyrophosphorsäure oder Polyphosphorsäure. Geeignete inerte Lösungsmittel sind beispielsweise Tetrachlormethan, Tetrachloräthan, Trichloräthylen, Schwefelkohlenstoff oder Nitrobenzol. Erforderlichenfalls arbeitet man unter Kühlen oder Erwärmen, z.B. bei etwa $-10$ bis etwa $+40°C$, insbesondere bei $+5$ bis $+30°C$.

Ausgangsstoffe II können z.B. hergestellt werden, indem man Verbindungen der Formeln

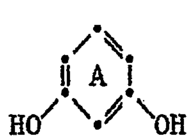 (XIII) und 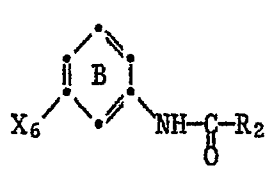 (VIII) ,

worin $X_6$ einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest, z.B. eine Gruppe der Formel

$$X'-CH_2-[-O-(CH_2)_n-]_o-O- \text{ bzw.}$$
$$X-[-(CH_2)_n-O-]_p-CH_2-CH(OH)-CH_2-[-O-(CH_2)n-]_o-O-$$

bedeutet, in der X reaktionsfähiges verestertes Hydroxy, z.B. Halogen, und X' Epoxyäthyl darstellt, in üblicher Weise miteinander umsetzt und das Reaktionsprodukt der Formel

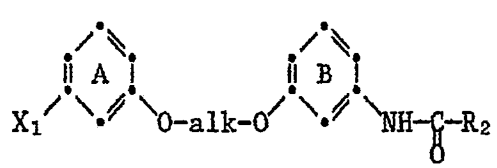 (III),

worin $X_1$ Hydroxy ist, in üblicher Weise, z.B. durch Umsetzung mit einer Verbindung $R_1-X_2$ (IV), worin $X_2$ z.B. Halogencarbonyl oder anhydridisiertes Carboxy der Formel $-C(=O)-O-(=O)-R_1$ bedeutet, O-acyliert. Die Herstellung von Verbindungen VIII ist unter der Verfahrensvariante e) beschrieben.

Die Umsetzung von Verbindungen III und IV gemäß Verfahrensvariante b) erfolgt üblicherweise in Gegenwart eines sauren Kondensationsmittels, vorteilhaft in einem inerten Lösungsmittel, erforderlichenfalls unter Kühlen oder Erwärmen, beispielsweise bei etwa $-10$ bis $+100°C$, vor allem bei $+5$ bis $+65°C$. Als saure Kondensationsmittel und inerte Lösungsmittel kommen beispielsweise die für die Verfahrensvariante a) angegebenen in Betracht.

Ausgangsstoffe III können beispielsweise hergestellt werden, indem man Verbindungen der Formeln

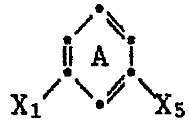 (XIV) und 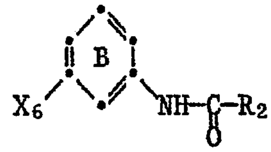 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls

durch Sauerstoff unterbrochenen Hydroxyniederalkoxyrest oder einen gegebenenfalls durch Sauerstoff unterbrochenen Epoxyniederalkoxyrest, z.B. eine Gruppe der Formel

$$X'-CH_2-[-O-(CH_2)_n-]_o-O- \text{ bzw.}$$

$$X-[-(CH_2)_{n'}-O-]_p-CH_2-CH(OH)-CH_2-[-O-(CH_2)_n-]_o-O-$$

bedeutet, in der X reaktionsfähiges verestertes Hydroxy, z.B. Halogen, und X' Epoxyäthyl darstellt.

Ausgangsstoffe IV sind bekannt.

Die Überführung von $X_3$ in eine Gruppe $R_1-C(=O)-$ gemäß der Verfahrensvariante c) erfolgt in üblicher Weise, ausgehend von Verbindungen V, worin $X_3$ eine Gruppe der Formel $R_1-CH(X_9)-$, beispielsweise durch Oxidation, ausgehend von Verbindungen V, worin $X_3$ eine Gruppe der Formel $R_1-C(=NH)-$ bedeutet, beispielsweise durch Solvolyse und ausgehend von Verbindungen V, worin $X_3$ gegebenenfalls funktionell abgewandeltes oder in einer Salzform vorliegendes Carboxy ist, beispielsweise durch Umsetzung mit einer Verbindung der Formel $R_1-M$ (XV), worin M einen metallischen Rest, z.B. der Formeln $-M^I$, $-M^{II}/2$ oder $M^{II}-Hal$, $-M^I$ ein Alkalimetallatom, z.B. Lithium, $M^{II}$ ein Erdalkali- oder Erdmetallatom, z.B. Magnesium, Zink oder Cadmium, und Hal ein Halogenatom, z.B. Chlor, Brom oder Iod, bedeutet.

Für die Oxidation von Gruppen $X_3$ der Formel $R_1-CH(X_9)-$ geeignete Oxidationsmittel sind beispielsweise oxidierende Schwermetallverbindungen, wie $Cr^{III}$-, $Cr^{VI}$-, $Mn^{IV}$-, $Mn^{VII}$-, $Fe^{III}$-, $Sn^{IV}$- oder $V^V$-Verbindungen, z.B. Chromtrioxid, Kaliumchromat bzw. -dichromat, Mangandioxid, Kaliumpermanganat, Eisentrichlorid, Zinntetrachlorid oder Vanadiumpentoxid, ferner oxidierende Sauerstoffsäuren des Stickstoffs, Wismuts, Selens oder der Halogene bzw. deren Salze oder Anhydride, wie Stickstoffdioxid, Wismutoxid, Selendioxid, Natriumhypochlorit, Kaliumchlorat oder Kaliumperiodat. Für die Oxidation von Gruppen $X_3$ der Formel $R_1-CH(X_9)-$, worin $X_9$ Hydroxy bedeutet, besonders geeignet ist z.B. Kaliumpermanganat in wäßrigem Pyridin oder wäßrigem Aceton bzw. Pyridiniumdichromat in Dichlormethan.

Die Solvolyse von Gruppen $X_3$ der Formel $R_1-C(=NH)-$ erfolgt insbesondere durch milde Hydrolyse, d.h. Einwirkung von Wasser, erforderlichenfalls in Gegenwart eines milden Hydrolysemittels. Als solche kommen beispielsweise Protonensäuren, wie Mineralsäuren, z.B. Chlorwasserstoffsäure oder Schwefelsäure, oder organische Sulfon- oder Carbonsäuren, wie Niederalkan- bzw. gegebenenfalls substituierte Benzolsulfonsäuren oder Niederalkansäuren, z.B. p-Toluolsulfonsäure oder Essigsäure, in Betracht.

Die Umsetzung von Verbindungen V, worin $X_3$ eine gegebenenfalls funktionell abgewandelte oder in einer Salzform vorliegende Carboxygruppe ist, mit einer Metallverbindung XV erfolgt in üblicher Weise, beispielsweise in einem Diniederalkyl- oder Alkylenäther, wie Diäthyläther, Tertiärbutyloxymethan, Dioxan oder Tetrahydrofuran, erforderlichenfalls unter Kühlen oder gelindem Erwärmen, z.B. bei −60 bis +40°C, vor allem bei 20 bis +25°C.

Ausgangsstoffe V können beispielsweise hergestellt werden, indem man Verbindungen der Formeln

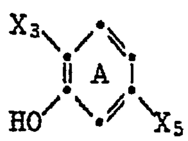 (XVI) und 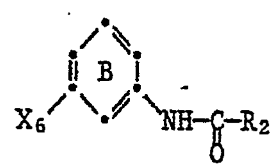 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest, z.B. eine Gruppe der Formel

$$X'-CH_2-[-O-(CH_2)_n-]_o-O- \text{ bzw.}$$

$$X-[-(CH_2)_{n'}-O-]_p-CH_2-CH(OH)-CH_2-[-O-(CH_2)_n-]_o-O-$$

bedeutet, in der X reaktionsfähiges verestertes Hydroxy, z.B. Halogen, und X' Epoxyäthyl darstellt.

Ausgangsstoffe V, worin $X_3$ eine Gruppe $R_1-CH(X_9)-$ bedeutet, können ferner hergestellt werden, indem man Verbindungen XVI ($X_3$ = Formyl) und VIII miteinander umsetzt und das Reaktionsprodukt V ($X_3$ = Formyl) mit einer Metallverbindung XV weiterumsetzt.

Ausgangsstoffe V, worin $X_3$ eine Gruppe $R_1-C(=NH)-$ bedeutet, können ferner hergestellt werden, indem man eine Verbindung der Formel

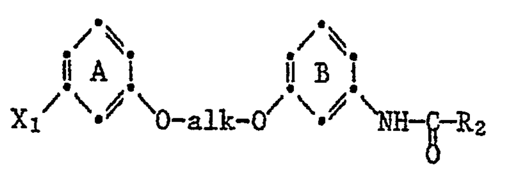 (III),

worin $X_1$ die eingangs angegebene Bedeutung hat und z.B. für Niederalkoxy steht, in Gegenwart einer Lewissäure, z.B. eines komplexen Metallhalogenides der Formel $M^nY_n$ (XIX), worin M, n und Y die für

die Verfahrensvariante a) angegebenen Bedeutungen haben, insbesondere von Aluminiumchlorid, mit einem Nitril der Formel $R_1$–CN (XVII) umsetzt. Das Zwischenprodukt V braucht dabei nicht isoliert zu werden; es reagiert unter den Aufarbeitungsbedingungen im allgemeinen erfindungsgemäß weiter.

Die Überführung von Gruppen $X_4$ in Hydroxy gemäß der Verfahrensvariante d) erfolgt in üblicher Weise, beispielsweise durch Behandeln mit einem komplexen Metallhalogenid der Formel $M^nY_n$ (XIX), worin M ein n-wertiges, koordinativ ungesättigtes Metallkation der Gruppe IIa, IIb, IIIa, IIIb, IVb, Va oder VIIIb des periodischen Systems der Elemente, z.B. ein Magnesium-, $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$- bzw. $Eisen^{VI}$-ion, und Y ein Halogenatom der Atomnummer bis und mit 35, wie Fluor oder Chlor, bedeutet, z.B. mit Aluminiumtrichlorid, oder mit einem tertiären organischen Ammoniumsalz, wie einem Pyridinium- oder Triniederalkylammoniumhalogenid, z.B. mit Pyridiniumchlorid oder -bromid oder Triäthylammoniumchlorid, kann aber auch durch Solvolyse, insbesondere durch Hydrolyse, erforderlichenfalls in Gegenwart eines, vorzugsweise sauren, Hydrolysemittels, erfolgen. Hydrolysemittel sind neben üblichen basischen Hydrolysemitteln, wie Alkalimetallhydroxiden, als saure Hydrolysemittel beispielsweise Mineralsäuren, z.B. Chlor-, Brom- oder Iodwasserstoffsäure, Schwefelsäure, Phosphorsäure oder Polyphosphorsäure, ebenso komplexe Metallsäuren, z.B. Hexachloroantimonsäure, Tetrafluorborsäure und dergleichen, im Falle von mit organischen Carbonsäuren veresterten Hydroxygruppen $X_4$ ferner Niederalkansäuren, wie Essigsäure. Lösungsmittel sind bei der Hydrolyse beispielsweise mit Wasser mischbare organische Lösungsmittel. Vorzugsweise arbeitet man jeweils in Gegenwart eines Lösungs- oder Verdünnungsmittels bzw. eines Lösungsvermittlers, unter Kühlen oder Erwärmen, z.B. im Temperaturbereich von etwa 0 bis 120°C, und/oder unter Inertgas.

So kann man verätherte Hydroxygruppen beispielsweise durch Behandeln mit wäßriger Iodwasserstoffsäure, Pyridiniumhydrochlorid, z.B. in Dichlormethan, mit Bromwasserstoffsäure in hochkonzentrierter, z.B. 98%iger, Essigsäure oder durch Behandeln mit Bortribromid oder Aluminiumtrichlorid zu Hydroxy spalten. In einer Abwandlung dieses Verfahrens kann man z.B. Verbindungen der Formeln

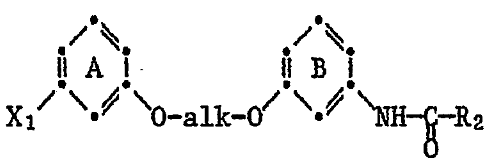

(III) und $R_1$-$X_2$ (IV) ,

worin $X_1$ veräthertes Hydroxy und $X_2$ anhydridisiertes Hydroxy, wie Halogen oder eine Gruppe der Formel $R_1$–C(=O)–O–, bedeutet, mit einer Lewissäure, wie mit einem komplexen Metallhalogenid der Formel $M^nY_n$ (XIX), z.B. Aluminiumtrichlorid, behandeln, wobei aus der primär gebildeten Verbindung VI, worin $X_4$ veräthertes Hydroxy bedeutet, die Hydroxygruppe freigesetzt wird. In einer anderen Abwandlung dieser Verfahrensvariante kann man eine Verbindung VI, worin $X_4$ Niederalk-2-enyloxy bedeutet und der Ring A in o-Stellung zu $X_4$ unsubstituiert ist, durch Erwärmen auf etwa 150 bis 250°C, vorzugsweise auf etwa 190 bis 220°C, vorteilhaft in einem Lösungsmittel, wie Diphenyläther, zu einer Verbindung I umlagern, worin der Ring A in o-Stellung zur Hydroxygruppe durch Niederalk-2-enyl substituiert ist.

In als Gruppe $X_4$ eine gegebenenfalls substituierte α-Phenylniederalkoxygruppe oder eine andere übliche durch Reduktion spaltbare geschützte Hydroxygruppe aufweisenden Verbindungen VI kann die Hydroxygruppe vorteilhaft reduktiv freigesetzt werden. So kann man beispielsweise hydrieren, d.h. mit Wasserstoff in Gegenwart eines Hydrierungskatalysators, z.B. eines Palladium-, Platin-, Nickel- oder Rhodiumkatalysators, z.B. von Palladium auf Kohle oder von Raney-Nickel, reduzieren.

Ferner kann man ausgehend von Verbindungen VI, worin $X_4$ mit einer organischen Carbonsäure verestertes Hydroxy ist, die Hydroxygruppe durch Umesterung, d.h. durch Behandlung mit einem Alkohol, z.B. Niederalkanol, in Gegenwart eines sauren oder basischen Mittels, wie einer Mineralsäure, z.B. von Schwefelsäure oder eines Alkalimetallhydroxides oder -alkoholates, z.B. von Natriumhydroxid oder eines Natriumniederalkanolates, freisetzen.

Ausgangsstoffe VI werden beispielsweise hergestellt, indem man Verbindungen der Formeln

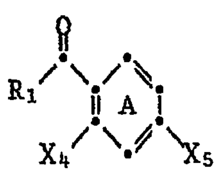

(XVIII) und

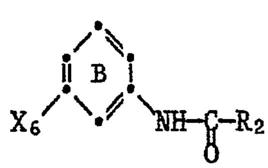

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest, z.B. eine Gruppe der Formel

$X'-CH_2-[-O-(CH_2)_n-]_o-O-$ bzw.

$X-[-(CH_2)_{n'}-O-]_p-CH_2-CH(OH)-CH_2-[-O-(CH_2)_n-]_o-O-$

bedeutet, in der X reaktionsfähiges verestertes Hydroxy, z.B. Halogen, und X' Epoxyäthyl darstellt, oder zur Herstellung von Verbindungen VI, worin $X_4$ veräthertes Hydroxy $X_1$ bedeutet, eine entsprechende Verbindung der Formel

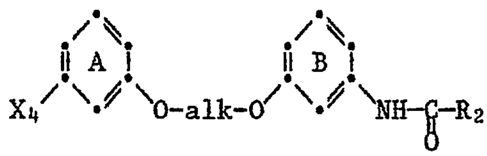

(IIIa)

unter schonenden Bedingungen, z.B. bei –10 bis +10°C oder unter Verwendung einer mildwirkenden Lewissäure, z.B. von Zinkchlorid oder Galliumchlorid, mit einer Verbindung der Formel $R_1-X_2$ [IV, $X_2$ = Halogencarbonyl oder $-O-C(=O)-R_1$] zur Reaktion bringt.

Die Umsetzung von Verbindungen VII und VIII gemäß <u>Verfahrensvariante e)</u> erfolgt in üblicher Weise, ausgehend von Ausgangsstoffen, worin $X_5$ bzw. $X_6$ einen durch reaktionsfähiges verestertes Hydroxy substituierten Rest –O–alkH bedeutet, beispielsweise in Gegenwart eines basischen Kondensationsmittels, wie eines Hydroxides oder Carbonates eines Alkali- oder Erdalkalimetalles, wie von Natrium- oder Kaliumhydroxid, Kaliumcarbonat oder Calciumcarbonat, vorteilhaft in einem Niederalkanol, z.B. Methanol oder Amylalkohol, Diniederalkylketon, z.B. in Aceton oder Diäthylketon, oder N,N-Diniederalkylniederalkansäureamid oder N-Niederalkylniederalkansäurelactam, z.B. in Dimethylformamid oder N-Methylpyrrolidinon.

Die Ausgangsstoffe VII werden beispielsweise hergestellt, indem man eine Verbindung der Formel

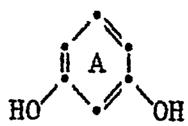

(XIII)

in Gegenwart einer Lewissäure, z.B. von Aluminiumtrichlorid oder Zinkchlorid, mit einer Verbindung der Formel $R_1-X_2$ [IV; $X_2$ = Halogencarbonyl oder $-O-C(=O)-R_1$] umsetzt und gewünschtenfalls in der erhaltenen Verbindung VII, worin $X_5$ Hydroxy ist, die Hydroxygruppe in p-Stellung zu $R_1-C(=O)-$ durch Umsetzung mit einem gegebenenfalls durch Sauerstoff unterbrochenen Dihalogenalkanol oder Halogenalkanepoxid in einen durch Halogen substituierten, gegebenenfalls durch Sauerstoff unterbrochenen Hydroxyalkoxyrest bzw. einen gegebenenfalls durch Sauerstoff unterbrochenen Epoxyalkoxyrest überführt.

Verbindungen VIII können beispielsweise erhalten werden, indem man in einer Verbindung der Formel

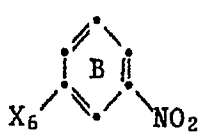

(XX)

die Nitrogruppe zu Amino reduziert, z.B. mit Wasserstoff in Gegenwart von Raney-Nickel, und die Verbindung der Formel

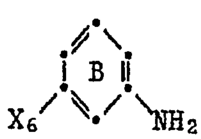

(XXI)

in Gegenwart einer Base, z.B. von Triäthylamin oder Pyridin, mit einer Verbindung der Formel $Hal-C(=O)-R_2$ (XIb; Hal = Halogen) umsetzt und gewünschtenfalls in der erhaltenen Verbindung VIII, worin $X_6$ Hydroxy ist, die Hydroxygruppe $X_6$ durch Umsetzung mit einem gegebenenfalls durch Sauerstoff unterbrochenen Dihalogenalkanol oder Halogenalkanepoxid in einen durch Halogen substituierten, gegebenenfalls durch Sauerstoff unterbrochenen Hydroxyalkoxyrest bzw. einen gegebenenfalls durch Sauerstoff unterbrochenen Epoxyalkoxyrest überführt.

Die Überführung von Hydroxy $X_5$ bzw. $X_6$ in einen durch reaktionsfähiges verestertes Hydroxy substituiertes, gegebenenfalls durch Sauerstoff unterbrochenen Hydroxyalkoxyrest oder einen gegebenenfalls durch Sauerstoff unterbrochenen Epoxyalkoxyrest kann dabei jeweils auch stufenweise durchgeführt werden. So kann man eine Verbindung VII bzw. VIII mit freier Hydroxygruppe $X_5$ bzw. $X_6$ zunächst mit einer Verbindung der Formel $X-(CH_2)_n-OH$ (XXII) zu der entsprechenden Verbindung VII bzw. VIII umsetzen, worin $X_5$ bzw. $X_6$ eine Gruppe der Formel

$$-O-[-(CH_2)_n-]_o-X$$

und o 1 bedeutet. Dieses Zwischenprodukt kann man dann a) mit einer Verbindung der Formel $X-CH_2-X'$ (XXIV) oder b) zunächst mit einer Verbindung XXIV und dann mit einer Verbindung XXII umsetzen, um zu einer entsprechenden Verbindung VII oder VIII zu gelangen, worin $X_5$ bzw. $X_6$ eine Gruppe der Formeln

a) $-O-[-(CH_2)_n-O-]_o-CH_2-X'$ oder

b) $-O-[-(CH_2)_n-O-]-CH_2-CH(OH)-CH_2-O-[-(CH_2)_n-]_p-X$

worin o und p für 1 stehen, bedeutet.

Man kann aber auch mit einer Verbindung der Formel $X-CH_2-CH(X'')-CH_2X$ (XXV), worin X reaktionsfähiges verestertes Hydroxy, wie Chlor, Brom oder Iod, und X" mit einer Carbonsäure verestertes Hydroxy, wie Niederalkanoyloxy, z.B. Acetoxy, bedeutet, zu einer Verbindung VII bzw. VIII, worin $X_5$ bzw. $X_6$ eine Gruppe der Formel $-O-CH_2-CH(X'')-CH_2X$ bedeutet, umsetzen und diese Gruppe durch Behandeln mit einem Alkalimetallhydroxid, z.B. mit Natriumhydroxid in Methanol, in einer Gruppe der Formel $-O-CH_2-X'$ überführen.

Die Überführung des Restes alk' einer Verbindung IX gemäß der Verfahrensvariante f) erfolgt in üblicher Weise, ausgehend von Verbindungen IX, worin alk' einen durch Oxo, mit einem α-Aralkanol oder einem Kohlensäuremono-α-aralkylester verestertes Hydroxy substituierten Niederalkylen- bzw. (Mono- oder Dioxa)niederalkylenrest bedeutet, beispielsweise reduktiv und ausgehend von Verbindungen IX, worin alk' einen durch andere verätherte oder veresterte Hydroxygruppen als die vorstehend genannten substituierten Niederalkylen- bzw. (Mono- oder Dioxa)niederalkylenrest bedeutet, solvolytisch.

Als Reduktionsmittel für die Reduktion von durch Oxo substituierten Resten alk' zu den entsprechenden Resten alk kommen beispielsweise Alkalimetallborhydride, wie Lithiumborhydrid, Natriumborhydrid oder Natriumcyanoborhydrid, oder sekundäre Alkohole, wie sekundäre Niederalkanole oder Cycloniederalkanole, z.B. Isopropanol oder Cyclohexanol, in Gegenwart eines Aluminiumalkoholats, insbesondere Isopropanol in Gegenwart von Aluminiumisopropanolat, in Betracht. Die Umsetzung mit den genannten Alkalimetallborhydriden wird vorteilhaft in einem Niederalkanol, einem Diniederalkyläther oder Niederalkylenäther oder Gemischen dieser Lösungsmittel, z.B. in Aethanol, durchgeführt. Bei der Umsetzung mit Alkoholen in Gegenwart eines Aluminiumalkoholats arbeitet man zweckmäßigerweise in einem Überschuß des als Reduktionsmittels verwendeten Alkohols.

Die reduktive Spaltung von α-Aralkoxy bzw. α-Aralkoxycarbonyloxy zu Hydroxy erfolgt vorzugsweise durch Hydrogenolyse, d.h. Einwirkung von Wasserstoff in Gegenwart eines Hydrierungskatalysators, wie eines Palladium-, Platin-, Iridium- oder Nickel-Katalysators, z.b. von Palladium auf Kohle, Platinoxid oder Raney-Nickel, vorteilhaft in einem Niederalkanol, z.B. in methanolischer Lösung, erforderlichenfalls unter Überdruck und/oder Erwärmen, z.B. bei etwa 1 bis 10 bar und 20 bis 60°C.

Die solvolytische Freisetzung von Hydroxy aus anderen als den genannten verätherten oder veresterten Hydroxygruppen, wie aus Silyloxy, Niederalkanoyloxy, erfolgt beispielsweise durch Hydrolyse (Umsetzung mit Wasser), Alkoholyse (Umsetzung mit einem Alkohol) oder Ammono- bzw. Aminolyse (Umsetzung mit Ammoniak oder einem mindestens ein freies Wasserstoffatom aufweisenden Amin), erforderlichenfalls in Gegenwart eines sauren oder basischen Mittels und/oder unter Erwärmen, z.B. bei etwa 20° bis 100°C. Geeignete saure Mittel sind beispielsweise Mineralsäuren, wie Halogenwasserstoffsäuren, z.B. Chlorwasserstoffsäure, oder Sauerstoffsäuren des Schwefels oder Phosphors, z.B. Schwefel- oder Phosphorsäure, oder organische Sulfon- oder Carbonsäuren, von Niederalkansulfonsäuren oder gegebenenfalls substituierte Benzolsulfonsäuren bzw. Niederalkansäuren, z.B. p-Toluolsulfonsäure oder Essigsäure. Basische Hydrolysemittel sind beispielsweise Hydroxide oder Carbonate von Alkalimetallen, z.B. Kaliumcarbonat, Natriumhydroxid oder Kaliumhydroxid, für die Alkoholyse ferner entsprechende Alkoholate, wie Alkalimetallniederalkanolate, z.B. Natriummethanolat.

Ausgangsstoffe IX können beispielsweise erhalten werden, indem man Verbindungen der Formeln

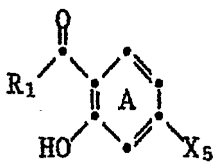 (VII) und 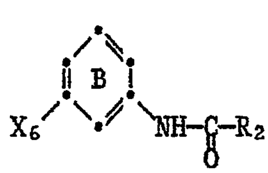 (VIII)

miteinander umsetzt, worin einer der Reste $X_5$ und $X_6$ gegebenenfalls in Salzform vorliegendes Hydroxy und der andere einen reaktionsfähiges verestertes Hydroxy X aufweisenden Rest $-O-alk'-X$, d.h. einen durch reaktionsfähiges verestertes Hydroxy und zusätzlich durch Oxo oder veräthertes bzw. verestertes Hydroxy substituierten, gegebenenfalls durch Sauerstoff unterbrochenen Alkoxyrest, bedeutet. Dieser hat beispielsweise eine der Formeln

$-O-[-(CH_2)_n-O-]_o-CH_2-C)=O)-CH_2-[-O-(CH_2)_{n'}-]_p-X$ oder

$-O-[-(CH_2)_n-O-]_o-CH_2-CH(X'')-CH_2-[-O-(CH_2)_{n'}-]_p-X$,

Die Umsetzung der entsprechenden Verbindungen VII und VIII sowie deren Herstellung erfolgt z.B. in analoger Weise wie für die Verfahrensvariante e) angegeben.

Die Umsetzung von Verbindungen X und XI gemäß Verfahrensvariante g) kann in üblicher, insbesondere in der aus der Literatur für analoge Umsetzungen bekannten, Weise erfolgen, erforderlichenfalls in Gegenwart eines Kondensationsmittels, bei der Umsetzung mit einem gegebenenfalls in 1-Stellung geschützten 5-Halogencarbonyltetrazol, beispielsweise eines basischen Kondensationsmittels, wie einer tertiären organischen Stickstoffbase, z.B. von Triäthylamin oder Pyridin.

In 1-Stellung geschützte 5-Halogencarbonyltetrazole werden vorzugsweise in situ hergestellt, z.B. durch Umsetzung eines Alkalimetall-, z.B. des Kaliumsalzes, einer in 1-Stellung geschützten, z.B. α-aralkylierten, Tetrazol-5-carbonsäure mit Oxalylchlorid in Gegenwart von Pyridin.

Die 1-Schutzgruppe von 5-Tetrazolylresten $R_2$ kann anschließend z.B. durch Behandeln mit Trifluoressigsäure/Anisol oder hydrogenolytisch, insbesondere mittels Wasserstoff und Palladium auf Kohle, abgespalten werden.

Ausgangsstoffe X können beispielsweise hergestellt werden, indem man Verbindungen der Formeln

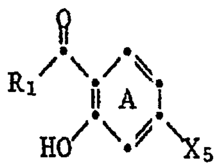 (VII) und 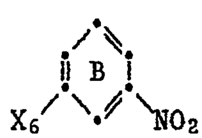 (XX)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest, z.B. eine Gruppe der Formel

$X'-CH_2-[-O-(CH_2)_n-]_o-O-$ bzw.

$X-[-(CH_2)_{n'}-O-]_p-CH_2-CH(OH)-CH_2-(CH_2)_n-]_o-O-$

bedeutet, worin X reaktionsfähiges verestertes Hydroxy, z.B. Halogen, und X' Epoxyäthyl darstellt, und in der erhaltenen Verbindung der Formel

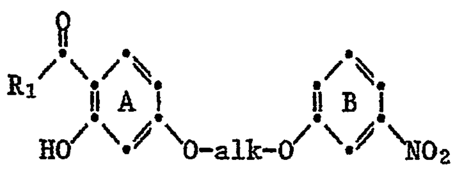 (XXVI)

die Nitrogruppe zu Amino reduziert, beispielsweise durch Umsetzung mit Wasserstoff in Gegenwart eines Hydrierungskatalysators, wie von Palladium auf Kohle oder vor allem Raney-Nickel, beispielsweise in Tetrahydrofuran.

Die Überführung der Gruppe $X_8$ in Verbindungen XII in solche der Formel $-NH-C(=O)-R_2$ gemäß Verfahrensvariante h) erfolgt beispielsweise durch Umsetzung mit Stickstoffwasserstoffsäure.

Die Umsetzung von Gruppen $X_8$ der Formel $-NH-C(=O)-CN$ mit Stickstoffwasserstoffsäure erfolgt vorzugsweise unter Bildung derselben in situ durch Behandeln eines Alkalimetallazides mit einer Säure, wie Chlorwasserstoffsäure, vorzugsweise in Toluol oder ähnlichen Lösungsmitteln.

Die Ausgangsstoffe XII werden beispielsweise hergestellt, indem man eine Verbindung der Formel

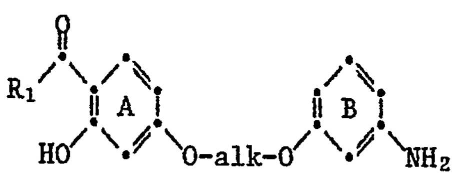 (X)

oder ein Säureadditionssalz davon mit Cyanameisensäure der Formel NC–COOH (XXVIIa) oder einem vorzugsweise funktionellen Derivat davon umsetzt. Funktionelle Derivate von Säuren XXVIIa sind vor allem eine veresterte oder anhydridisierte Carboxygruppe, wie Niederalkoxycarbonyl oder Halogencarbonyl, z.B. Chlor- oder Bromcarbonyl, enthaltende Säurederivate. Als funktionelle Derivate von Säuren XXVIIa seien z.B. Cyanoformylchlorid oder Cyanogen genannt.

Die Umsetzung von Verbindungen X mit Säuren XXVIIa bzw. deren Derivaten kann in üblicher Weise erfolgen, beispielsweise in Gegenwart eines wasserbindenden Mittels, wie eines Säureanhydrides, z.B. von Phosphorpentoxid, oder von Dicyclohexylcarbodiimid, oder eines z.B. sauren oder basischen Kondensationsmittels, wie einer Mineralsäure, z.B. von Chlorwasserstoffsäure, oder eines Alkalimetallhydroxides oder -carbonates, z.B. von Natrium- oder Kaliumhydroxid, oder einer organischen Stickstoffbase, z.B. von Triäthylamin oder Pyridin. Bei der Umsetzung mit einem Säureanhydrid, wie Säurechlorid, verwendet man vorzugsweise eine organische Stickstoffbase als Kondensationsmittel. Die Umsetzung mit Carbonsäuren führt man vorzugsweise in Gegenwart eines wasserbindenden Mittels durch. Erforderlichenfalls arbeitet man jeweils in einem inerten Lösungsmittel, bei normaler Temperatur oder unter Kühlen oder Erwärmen, z.B. in einem Temperaturbereich von etwa 0° bis etwa 100°C, in einem geschlossenen Gefäß und/oder unter Inertgas, z.B. Stickstoff.

Eine erfindungsgemäß erhältliche Verbindung der allgemeinen Formel I kann in an sich bekannter Weise in eine andere Verbindung der allgemeinen Formel I umgewandelt werden.

So kann man beispielsweise eine freie Carboxylgruppe in üblicher Weise, z.B. durch Behandeln mit einem Diazoniederalkan oder Triniederalkyloxonium-, Triniederalkylcarboxonium- oder Diniederalkylcarboniumsalz, wie -hexachloroantimonat oder -hexafluorophosphat, oder vor allem durch Umsetzung mit einem Niederalkanol oder einem reaktionsfähigen Derivat, wie einem Carbon-, Phosphorig-, Schweflig- oder Kohlensäureester davon, z.B. Niederalkancarbonsäureester, Triniederalkylphosphit oder Diniederalkylsulfit, in Niederalkoxycarbonyl oder durch Umsetzung mit Ammoniak, Dehydratisierung der primär gebildeten Ammoniumsalzform und gewünschtenfalls Carbamylgruppe in Carbamyl oder Cyano überführen.

Niederalkoxycarbonyl, Carbamyl oder Cyano kann in üblicher Weise, z.B. durch Hydrolyse in Gegenwart eines Katalysators, beispielsweise eines basischen oder sauren Mittels, wie einer starken Base, z.B. Natrium- oder Kaliumhydroxid, oder einer Mineralsäure, z.B. von Salzsäure, Schwefelsäure oder Phosphorsäure, zur freien Carboxylgruppe hydrolysiert werden.

In einer erfindungsgemäß erhältlichen Verbindung kann man weiterhin durch Umsetzung mit einem Niederalkylhalogenid oder Niederalken bzw. einem Niederalkansäurehalogenid oder -anhydrid, jeweils in Gegenwart einer Lewissäure, wie Aluminiumtrichlorid, in den Ring A und/oder B Niederalkyl bzw. in den Ring B Niederalkanoyl einführen. Ferner kann man Halogen einführen, beispielsweise durch Behandeln mit einem Halogen in Gegenwart einer Lewissäure, wie Eisen-III-chlorid, oder durch Umsetzung mit N-Chlorsuccinimid. Ferner kann man Niederalkenyl- bzw. Niederalkinylreste zu Niederalkyl reduzieren, beispielsweise durch Behandeln mit Wasserstoff in Gegenwart eines Hydrierungskatalysators, z.B. von Palladium auf Kohle. Weiterhin kann man Halogen, insbesondere Iod, durch Umsetzung mit Trifluoriodmethan in Gegenwart von Kupfer durch Trifluormethyl bzw. durch Umsetzung mit einem Alkalimetallcyanid durch Cyano ersetzen.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden, oder als Isomerengemische, wie Racemate, Diastereoisomerengemische oder Racematgemische, vorliegen.

Erhaltene Diastereomerengemische und Racematgemische können aufgrund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzen eines sauren Endstoffes mit einer mit der racemischen Säure Salze bildenden optisch aktiven Base und Trennung der auf diese Weise erhaltenen Salze, z.B. aufgrund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Erhaltene freie Verbindungen der Formel I können in an sich bekannter Weise in Salze überführt werden, z.B. durch Behandeln mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen der Formel I umgewandelt werden, z.B. durch Behandeln mit einem sauren Reagens, wie einer Mineralsäure.

Die Verbindungen der Formel I einschließlich ihrer Salze können auch in Form ihrer Hydrate erhalten werden oder das zur Kristallisation verwendete Lösungsmittel einschließen.

Infolge der engen Beziehung zwischen den neuen Verbindungen der Formel I in freier Form und in Form ihrer Salze sind im vorausgegangenen und nachfolgend unter den freien Verbindungen der Formel I oder ihren Salzen sinn- und zweckgemäß gegebenenfalls auch die entsprechenden Salze bzw. freien Verbindungen der Formel I zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und

die fehlenden Schritte durchführt oder einen Ausgangsstoff in Form eines Salzes und/oder Racemates bzw. Antipoden verwendet oder insbesondere unter den Reaktionsbedingungen bildet.

Die bei dem erfindungsgemäßen Verfahren und seinen Vorstufen auftretenden neuen Ausgangsstoffe und Zwischenprodukte bilden ebenfalls einen Gegenstand der Erfindung.

Vorzugsweise werden solche Ausgangsstoffe verwendet und die Reaktionsbedingungen so gewählt, daß man zu den vorstehend als besonders bevorzugt aufgeführten Verbindungen gelangt.

Die vorliegende Erfindung betrifft ebenfalls pharmazeutische Präparate, welche eine der erfindungsgemäßen Verbindungen der Formel I oder ein pharmazeutisch verwendbares Salz davon enthalten. Bei den erfindungsgemäßen pharmazeutischen Präparaten handelt es sich um solche, die zur topischen und lokalen sowie enteralen, wie oralen oder rektalen, sowie parenteralen Verabreichung an und zur Inhalation durch Warmblüter bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand sowie von der Applikationsweise ab.

Die neuen pharmazeutischen Präparate enthalten z.B. von etwa 10% bis etwa 95%, vorzugsweise von etwa 20% bis etwa 90% des Wirkstoffs. Erfindungsgemäße pharmazeutische Präparate sind z.B. solche in Aerosol- oder Sprayform oder in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Werkstoff mit festen Trägerstoffen kombiniert, ein erhaltenes Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. Mais-, Weizen-, Reis- oder Kartoffelstärkekleister, Gelatine, Tragaganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fließregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Überzügen versehen, wobei man u.a. konzentrierte Zuckerlösungen, welche gegebenenfalls arabischen Gummi, Talk, Polyvinylpyrrolidone, Polyäthylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten:.als Grundmassenstoffe kommen z.B. flüssige Triglyceride, Polyäthylenglykole oder Paraffinkohlenwasserstoffe in Frage.

Zur parenteralen Verabreichung eignen sich in erster Linie wäßrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Aethyloleat oder Triglyceride, verwendet, oder wäßrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften erhalten außer dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder Verdünnungsmittel. Präparate, in welchen der pharmakologische Wirkstoff in Lösung vorliegt, enthalten außer diesem ein geeignetes Treibmittel, ferner, falls notwendig, ein zusätzliches Lösungsmittel und/oder einen Stabilisator. Anstelle des Treibgases

kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z.B. für die Behandlung der Haut Lotionen und Cremen, die eine flüssige oder semifeste Öl-in-Wasser- oder Wasser-in-Öl-Emulsion enthalten, und Salben (wobei solche vorzugsweise ein Konservierungsmittel enthalten), für die Behandlung der Augen Augentropfen, welche die aktive Verbindung in wäßriger oder öliger Lösung enthalten, und Augensalben, die vorzugsweise in steriler Form hergestellt werden, für die Behandlung der Base Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wäßriger öliger Lösung enthalten, oder für die lokale Behandlung des Mundes Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Die Erfindung betrifft ebenfalls die Verwendung der neuen Verbindungen der Formel I und ihrer Salze als pharmakologisch aktive Verbindungen, insbesondere als Antiallergika, vorzugsweise in der Form von pharmazeutischen Präparaten. Die tägliche Dosis, die einem Warmblüter von etwa 70 kg verabreicht wird, beträgt von etwa 200 mg bis etwa 1200 mg.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: Eine von 12.8 g (19.1 mMol) N-{3-[-(4-Acetyl-3-hydroxy-2-n-propyl-phenoxy)-2-hydroxy-propyloxy]-4-brom-6-methyl-phenyl}-1-(4-methoxybenzyl)-1H-tetrazol-5-carboxamid in 250 ml Trifluoressigsäure und 25 ml Anisol wird 30 Minuten zum Rückfluß erhitzt. Das Reaktionsgemisch wird unter vermindertem Druck eingeengt, mit etwa 300 ml Äther sowie 400 ml Petroläther versetzt und die Kristalle abfiltriert. Das so erhaltene N-{3-[3-(4-Acetyl-3-hydroy-2-n-propyl-phenoxy)-2-hydroxy-propyloxy]-4-brom-6-methyl-phenyl}-1H-tetrazol-5-carboxamid vom Smp. 155–158°; 1H-NMR-Spektrum (δ gegen TMS, DMSO-$d_6$): 0,85 (Triplett, 3H); 1,45 (Multiplett, 2H); 2,25 (Singulett, 3H); 2,55 (Multiplett, 2H); 2,60 (Singulett, 3H); 4,0–4,7 (Multiplett, 4H); 5,90 (Multiplett, 1H); 6,65 (Dublett, 1H); 7,30 (Singulett, 1H); 7,55 (Singulett, 1H); 7,85 (Dublett, 1H); 10,75 (Singulett, 1H); 12,80 (Singulett, 1H); wird in 100 ml Aceton gelöst und mit einem Äquivalent Triäthanolamin in 10 ml Aceton versetzt. Nach Zugabe von Äther setzt Kristallisation ein. Man erhält so das Triäthanolammoniumsalz von N-{3-[3-(4-Acetyl-3-hydroxy-2-n-propyl-phenoxy)-2-hydroxy-propyloxy]-4-brom-6-methyl-phenyl}-1H-tetrazol-5-carboxamid vom Smp. 76° (Zers.).

In analoger Weise erhält man ausgehend von N-{3-[3-(4-Acetyl-3-hydroxy-2-n-propyl-phenoxy)-2-hydroxy-propyloxy]-4-chlor-6-methyl-phenyl}-1-(4-methoxybenzyl)-1H-tetrazol-5-carboxamid das N-{3-[3-(4-Acetyl-3-hydroxy-2-n-propyl-phenoxy)-2-hydroxy-propyloxy]-4-chlor-6-methyl-phenyl}-1H-tetrazol-5-carboxamid, Öl; 1H-NMR-Spektrum (δ gegen TMS, DMSO-$d_6$): 0,85 (Triplett, 3H); 1,45 (Multiplett, 2H); 2,25 (Singulett, 3H); 2,55 (Multiplett, 2H); 2,60 (Singulett, 3H); 4,0–4,7 (Multiplett, 4H); 5,90 (Multiplett, 1H); 6,65 (Dublett, 1H); 6,65 (Dublett, 1H); 7,25 (Singulett, 1H); 12,80 (Singulett, 1H); und dessen Triäthanolammoniumsalz.

Das Ausgangsmaterial kann man z.B. wie folgt herstellen:

Eine Lösung von 16.2 g (70 mMol) 2-Brom-4-methyl-5-nitro-phenol in 130 ml Aethanol wird mit 0.32 g (7.4 mMol) einer 55%igen Natriumhydrid-Dispersion in Öl versetzt. Unter Rückfluß wird anschließend innerhalb von zwei Stunden eine Lösung von 18.4 g (73.5 mMol) 4-(2,3-Epoxypropoxy)-2-hydroxy-3-propyl-acetophenon in 150 ml Aethanol zugetropft. Danach erhitzt man noch weitere 8 Stunden zum Rückfluß. Das Reaktionsgemisch wird abgekühlt, im Vakuum auf ca. 100 ml eingeengt und auf Eis gegossen. Die wäßrige Phase wird mit verdünnter Salzsäure angesäuert und dreimal mit Dichlormethan extrahiert. Die vereinten organischen Phasen werden mit Wasser gewaschen, über Natriumsulfat getrocknet und im Vakuum eingedampft. Kristallisation aus Äther ergibt das 3-[3-(4-Acetyl-3-hydroxy-2-n-propyl-phenoxy)-2-hydroxy-propyloxy]-4-brom-6-methyl-nitrobenzol vom Smp. 104–106°.

In analoger Weise erhält man 3-[3-(4-Acetyl-3-hydroxy-2-n-propyl-phenoxy)-2-hydroxy-propyloxy]-4-chlor-6-methyl-nitrobenzol vom Smp. 103–105°C (ausgehend vom 2-Chlor-4-methyl-5-nitro-phenol).

Eine Lösung von 23.3 g (48.3 mMol) 3-[3-(4-Acetyl-3-hydroxy-2-n-propyl-phenoxy)-2-hydroxy-propyloxy]-4-brom-6-methyl-nitrobenzol in 240 ml Tetrahydrofuran wird mit 2.0 g Raney-Nickel versetzt und bei Raumtemperatur hydriert. Der Katalysator wird abfiltriert und mit Tetrahydrofuran gewaschen. Die vereinigten Filtrate werden im Vakuum eingeengt, mit Äther versetzt und die ausgefallenen Kristalle abfiltriert. Man erhält so 3-[3-(4-Acetyl-3-hydroxy-2-n-propyl-phenoxy)-2-hydroxy-propyloxy]-4-brom-6-methyl-anilin vom Smp. 78–80°.

In analoger Weise erhält man ausgehend von 3-[3-(4-Acetyl-3-hydroxy-2-n-propylphenoxy)-2-hydroxy-propyloxy]-4-chlor-6-methyl-nitrobenzol 3-[3-(4-Acetyl-3-hydroxy-2-n-propyl-phenoxy)-2-hydroxy-propyloxy]-4-chlor-6-methyl-anilin vom Smp. 94–95°.

Zu einer Suspension von 10.6 g (38.8 mMol) Kalium-[1-(4-methoxybenzyl)-1H-tetrazol]-5-carboxylat in 170 ml Benzol und 1.5 ml Pyridin gibt man bei 0–5° 3.38 ml (38.8 mMol) Oxalylchlorid und läßt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in

135 ml Dichlormethan gelöst und bei 0–5° innerhalb von 10 Minuten zu einer Lösung von 12.85 g (28.4 mMol) 3-[3-(4-Acetyl-3-hydroxy-2-n-propyl-phenoxy)-2-hydroxy-propyloxy]-4-brom-6-methyl-anilin und 2.7 ml (33 mMol) Pyridin in 100 ml Dichlormethan zugetropft. Anschließend wird 3 Stunden bei Raumtemperatur gerührt. Das Reaktionsgemisch wird mit Dichlormethan verdünnt und dreimal mit Wasser gewaschen. Die organische Phase wird über Natriumsulfat getrocknet und unter vermindertem Druck eingedampft. Der Rückstand wird mit Dichlormethan/Aethylacetat (6:1) über 400 g Kieselgel filtriert. Das Eluat wird eingedampft und der Rückstand aus Aethanol kristallisiert. Man erhält so das N-{3-[3-(4-Acetyl-3-hydroxy-2-n-propyl-phenoxy)-2-hydroxy-propyloxy]-4-brom-6-methyl-phenyl}-1-(4-methoxybenzyl)-1H-tetrazol-5-carboxamid vom Smp. 114–116°.

In analoger Weise erhält man ausgehend von 3-[3-(4-Acetyl-3-hydroxy-2-n-propyl-phenoxy)-2-hydroxy-propyloxy]-4-chlor-6-methyl-anilin N-{3-[3-(4-Acetyl-3-hydroxy-2-n-propyl-phenyl)-2-hydroxy-propyloxy]-4-chlor-6-methyl-phenyl}-1-(4-methoxybenzyl)-1H-tetrazol-5-carboxamid.

Beispiel 2: Eine Lösung von 7.1 g (11.8 mMol) N-{3-[3-(4-Acetyl-3-hydroxy-2-n-propyl-phenoxy)-2-hydroxy-propyloxy]-2-cyano-phenyl}-1-(4-methoxybenzyl)-1H-tetrazol-5-carboxamid in 150 ml Trifluoressigsäure und 15 ml Anisol wird 30 Minuten zum Rückfluß erhitzt. Das Reaktionsgemisch wird unter vermindertem Druck eingeengt, mit etwa 200 ml Äther sowie 300 ml Petroläther versetzt und die Kristalle abfiltriert. Das so erhaltene N-{3-[3-(4-Acetyl-3-hydroxy-2-n-propyl-phenoxy)-2-hydroxy-propyloxy]-2-cyano-phenyl}-1H-tetrazol-5-carboxamid vom Smp. 210–213° wird in 50 ml Aceton heiß gelöst und mit einem Äquivalent Triäthanolamin in 20 ml Aceton versetzt. Nach Zugabe von Äther setzt Kristallisation ein. Man erhält so das Triäthanolammoniumsalz von N-{3-[3-(4-Acetyl-3-hydroxy-2-n-propyl-phenoxy)-2-hydroxy-propyloxy]-2-cyano-phenyl}-1H-tetrazol-5-carboxamid vom Smp. 80–82°.

Die Ausgangsmaterialien erhält man beispielsweise wie folgt:

Zur Lösung von 34 g (207 mMol) 2-Hydroxy-6-nitro-benzonitril und 60 g 4-[(2,3-Epoxy)propyloxy]-2-hydroxy-3-propyl-acetophenon in 450 ml Dimethylformamid gibt man 30 Tropfen Triton B. Die Lösung wird 1,5 Stunden zum Rückfluß erhitzt, vom Lösungsmittel befreit und der Rückstand in 500 ml Aethylacetat aufgenommen. Nach Waschen mit 500 ml 0,5-n-Natronlauge und mit 500 ml Wasser über Magnesiumsulfat getrocknet. Das Lösungsmittel wird abgedampft und der Rückstand mit Methylenchlorid/Aethylacetat (95:5) an Kieselgel chromatographiert. Man erhält 2-[3-(4-Acetyl-3-hydroxy-2-propyl-phenoxy)-2-hydroxy-propoxy]-6-nitro-benzonitril vom Smp. 117–119°.

Zur Lösung von 10 g (24.2 mMol) 2-[3-(4-Acetyl-3-hydroxy-2-propyl-phenoxy)-2-hydroxy-propoxy]-6-nitro-benzonitril und 10 g Cyclohexen in 500 ml Aethanol gibt man 2.5 g 10% Palladium auf Kohle und erhitzt 30 Minuten zum Rückfluß. Nach Abkühlen auf Raumtemperatur wird filtriert und das Lösungsmittel abgedampft. Der Rückstand wird mit Äther versetzt, und die ausgeschiedenen Kristalle werden abfiltriert. Man erhält 3-[3-(4-Acetyl-3-hydroxy-2-n-propyl-phenoxy)-2-hydroxy-propyloxy]-2-cyano-anilin vom Smp. 123–125°.

Zu einer Suspension von 5.8 g (21.5 mMol) Kalium-{1-(4-methoxybenzyl)-1H-tetrazol}-carboxylat in 110 ml Benzol und 1.0 ml Pyridin gibt man bei 0–5° 1.84 ml (21.5 mMol) Oxalylchlorid und läßt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 80 ml Methylenchlorid gelöst und bei 0–5° innerhalb von etwa 10 Minuten zu einer Lösung von 5.87 g (17.2 mMol) 3-[3-(4-Acetyl-3-hydroxy-2-n-propyl-phenoxy)-2-hydroxy-propyloxy]-3-cyano-anilin und 1.72 ml (21.5 mMol) Pyridin in 40 ml Methylenchlorid zugetropft. Anschließend wird 3 Stunden bei Raumtemperatur gerührt. Das Reaktionsgemisch wird mit Methylenchlorid verdünnt und dreimal mit Wasser gewaschen. Die organischen Phasen werden vereinigt, über Natriumsulfat getrocknet und unter vermindertem Druck eingedampft. Der Rückstand wird mit Methylenchlorid/Essigsäureäthylester (6:1) an Kieselgel chromatographiert. Man erhält N-{3-[3-(4-Acetyl-3-hydroxy-2-n-propyl-phenoxy)-2-hydroxypropoxy]-2-cyano-phenyl}-1-(4-methoxybenzyl)-1H-tetrazol-5-carboxamid als farbloses Öl.

Beispiel 3: In analoger Weise wie in Beispiel 1 bzw. 2 beschrieben erhält man ausgehend von 2-Fluor-4-methyl-5-nitro-phenol, 4-Methyl-5-nitro-phenol bzw. 2-Hydroxy-5-methyl-4-nitro-benzonitril N-{3-[3-(4-Acetyl-3-hydroxy-2-n-propyl-phenoxy)-2-hydroxy-propyloxy]-4-fluor-6-methyl-phenyl}-1H-tetrazol-5-carboxamid 1H-NMR-Spektrum (δ gegen TMS, DMSO-$d_6$): 0,85 (Triplett, 3H); 1,45 (Multiplett, 2H); 2.25 (Singulett, 3H); 2.55 (Multiplett, 2H); 2,60 (Singulett, 3H); 4,0–4,7 (Multiplett, 4H); 5,90 (Multiplett, 1H); 6,65 (Dublett, 1H); 7,25 (Singulett, 1H); 7,65 (Singulett, 1H); 7,85 (Dublett, 1H); 10,70 (Singulett, 1H); 12,80 (Singulett, 1H); und sein Triäthanolammoniumsalz, N-{3-[3-(4-Acetyl-hydroxy-2-n-propyl-phenoxy)-2-hydroxy-propyloxy]-6-methyl-phenyl}-1H-tetrazol-5-carboxamid 1H-NMR-Spektrum (δ gegen TMS, DMSO-$d_6$): 0,85 (Triplett, 3H); 1,45 (Multiplett, 2H); 2,25 (Singulett, 3H); 2,55 (Multiplett, 2H); 2,60 (Singulett, 3H); 4,0–4,7 (Multiplett, 4H); 5,90 (Multiplett, 1H); 6,60 (Multiplett, 2H); 7,10 (Dublett, 1H); 7,80 (Multiplett, 2H); 10,70 (Singulett, 1H); 12,80 (Singulett, 1H); und sein Triäthanolammoniumsalz sowie N-{3-[3-(4-Acetyl-3-hydroxy-2-n-propyl-phenoxy)-2-hydroxy-propyloxy]-4-cyano-6-methyl-phenyl}-1H-tetrazol-5-carboxamid 1H-NMR-Spektrum (δ gegen TMS, DMSO-$d_6$): 0,85 (Triplett, 3H); 1,45 (Multiplett, 2H); 2,25 (Singulett, 3H); 2,55 (Multiplett, 2H); 2,60 (Singulett, 3H); 4,0–4,7 (Multiplett, 4H); 5,90 (Multiplett, 1H); 6,65 (Dublett, 1H); 7,30 (Singulett, 1H); 7,45 (Singulett, 1H); 7,85 (Dublett, 1H); 10,70 (Singulett, 1H); 12,80 (Singulett, 1H); und sein Triäthanolammoniumsalz.

Beispiel 4: 4.9 g N-{3-[3-(4-Acetyl-3-hydroxy-2-n-propyl)-phenoxy)-2-hydroxy-propyloxy]-6-methyl-phenyl}-1H-tetrazol-5-carboxamid werden in 45 ml Aceton gelöst und mit einer Lösung von 1.0 g Di-

äthanolamin in 5 ml Aceton versetzt. Nach Zugabe von Diäthyläther setzt Kristallisation ein. Man läßt 30 Minuten bei 5–10° stehen, saugt ab, wäscht mit Diäthyläther nach und läßt an der Luft trocknen. Man erhält das Diäthanolammoniumsalz von N-{3-[3-(4-Acetyl-3-hydroxy-2-n-propyl-phenoxy)-2-hydroxy-propyloxy]-6-methyl-phenyl}-1H-tetrazol-5-carboxamid. In analoger Weise kann man auch dessen Natrium-, Kalium-, Diäthylammonium- und Tris(hydroxymethyl)methylammoniumsalz herstellen.

In analoger Weise erhält man ferner jeweils das Natrium-, Kalium-, Diäthanolammonium-, Diäthylammonium- und Tris(hydroxymethyl)methylammoniumsalz von N-{3-[3-(4-Acetyl-3-hydroxy-2-n-propyl-phenoxy)-2-hydroxy-propyloxy]-2-cyano-phenyl}-1H-tetrazol-5-carboxamid, N-{3-[3-(4-Acetyl-3-hydroxy-2-n-propyl-phenoxy)-2-hydroxy-propyloxy]-4-brom-6-methyl-phenyl}-1H-tetrazol-5-carboxamid, N-{3-[3-(4-Acetyl-3-hydroxy-2-n-propyl-phenoxy)-2-hydroxy-propyloxy]-4-chlor-6-methyl-phenyl}-1H-tetrazol-5-carboxamid, N-{3-[3-(4-Acetyl-3-hydroxy-n-propyl-phenoxy)-2-hydroxy-propyloxy]-4-fluor-methyl-phenyl}-1H-tetrazol-5-carboxamid und N-{3-[-(4-Acetyl-3-hydroxy-2-n-propyl-phenoxy)-2-hydroxy-propyloxy]-4-cyano-6-methyl-phenyl}-1H-tetrazol-5-carboxamid.

Beispiel 5: Tabletten, enthaltend 25 mg Wirkstoff, z.B. das Triäthanolammoniumsalz von N-{3-[3-(4-Acetyl-3-hydroxy-2-propyl-phenoxy)-2-hydroxy-propyloxy]-4-brom-6-methyl-phenyl}-1H-tetrazol-5-carboxamid, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredenzien werden zunächst durch ein Sieb mit 0,6 mm Maschenweite getrieben. Dann werden der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpreßt.

In analoger Weise können auch Tabletten, enthaltend jeweils 25 mg einer anderen der in den Beispielen 1 bis 4 genannten Verbindungen der Formel I, hergestellt werden.

Beispiel 6: Kautabletten, enthaltend 30 mg Wirkstoff, z.B. das Triäthanolammoniumsalz von N-{3-[3-(4-Acetyl-3-hydroxy-2-propyl-phenoxy)-2-hydroxy-propyloxy]-4-brom-6-methyl-phenyl}-1H-tetrazol-5-carboxamid, können z.B. folgendermassen hergestellt werden:

| Zusammensetzung (für 1000 Tabletten) | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%-ige Gelatinelösung | q.s. |

Herstellung: Alle festen Ingredenzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpreßt.

In analoger Weise können auch Tabletten, enthaltend jeweils 30 mg einer anderen der in den Beispielen 1 bis 4 genannten Verbindungen der Formel I, hergestellt werden.

Beispiel 7: Tabletten, enthaltend 100 mg Wirkstoff, z.B. das Triäthanolammoniumsalz von N-{3-[3-(4-Acetyl-3-hydroxy-2-propyl-phenoxy)-2-hydroxy-propyloxy]-4-brom-6-methyl-phenyl}-1H-tetrazol-5-carboxamid, können folgendermassen hergestellt werden:

| Zusammensetzung (für 1000 Tabletten) | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredenzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpreßt.

In analoger Weise können auch Tabletten, enthaltend jeweils 100 mg einer anderen Verbindung der Formel I gemäß den Beispielen 1 bis 4, hergestellt werden.

Beispiel 8: Eine treibmittelhaltige, feststoffaerosolbildende Inhalationssuspension, enthaltend 0,1 Gew.-% N-{3-[3-(4-Acetyl-3-hydroxy-2-propyl-phenoxy)-2-hydroxy-propyloxy]-4-brom-6-methyl-phenyl}-1H-tetrazol-5-carboxamid (Wirkstoff), kann z.B. folgendermassen hergestellt werden:

| Zusammensetzung | |
|---|---|
| Wirkstoff, mikronisiert | 0,1 Gew.-% |
| "Sorbitantrioleate" | 0,5 Gew.-% |
| Treibmittel A (Trichlortrifluoräthan) | 4,4 Gew.-% |
| Treibmittel B (Gemisch aus 15 Teilen Dichlordifluormethan und 80 Teilen symm. Dichlortetrafluoräthan) | q.s. |

Herstellung: Der Wirkstoff wird unter Feuchtigkeitsausschluß mit Hilfe eines üblichen Homogenisators unter Zusatz des Sorbitantrioleats in dem Trichlortrifluoräthan suspendiert, die Suspension in einen Dosieraerosolbehälter abgefüllt, dieser verschlossen und unter Druck mit dem Dichlordifluormethan-Dichlortetrafluoräthan-Gemisch aufgefüllt.

In analoger Weise können auch Inhalationssuspensionen, enthaltend jeweils eine andere Verbindung der Formel I gemäß den Beispielen 1 bis 4, hergestellt werden.

Beispiel 9: Eine zur Inhalation geeignete, etwa 2%ige wäßrige Lösung, enthaltend das Triäthanolammoniumsalz von N-{3-[3-(4-Acetyl-3-hydroxy-2-propyl-phenoxy)-2-hydroxy-propyloxy]-4-brom-6-methyl-phenyl}-1H-tetrazol-5-carboxamid als Wirkstoff, kann z.B. in folgender Zusammensetzung hergestellt werden:

| Zusammensetzung | |
|---|---|
| Wirkstoff | 2000 mg |
| Stabilisator, z.B. Aethylendiamintetra-essigsäure-dinatriumsalz | 10 mg |
| Konservierungsmittel, z.B. Benzalkoniumchlorid | 10 mg |
| Wasser, frisch destilliert | ad 100 ml |

Herstellung: Der Wirkstoff wird in frisch destilliertem Wasser gelöst. Dann werden der Stabilisator und das Konservierungsmittel hinzugegeben. Nach vollständiger Auflösung aller Komponenten wird die

erhaltene Lösung auf 100 ml aufgefüllt und in Fläschchen abgefüllt. Diese werden gasdicht verschlossen.

In analoger Weise können auch 2%-ige Inhalationslösungen, enthaltend jeweils einen anderen Wirkstoff eines der Beispiele 1 bis 4, hergestellt werden.

Beispiel 10: Zur Insufflation geeignete, etwa 25 mg des Triäthanolammoniumsalzes von N-{3-[3-(4-Acetyl-3-hydroxy-2-propyl-phenoxy)-2-hydroxy-propyloxy]-4-brom-6-methyl-phenyl}-1H-tetrazol-5-carboxamid als Wirkstoff enthaltende, Kapseln können z.B. in folgender Zusammensetzung hergestellt werden:

| Zusammensetzung | |
|---|---|
| Wirkstoff | 25 g |
| Lactose, fein gemahlen | 25 g |

Herstellung: Der Wirkstoff und die Lactose werden innig vermischt. Das erhaltene Pulver wird sodann gesiebt und in Portionen zu je 50 mg in 1000 Gelatinekapseln abgefüllt.

In analoger Weise können auch Insufflationskapseln, enthaltend jeweils einen anderen Wirkstoff gemäß einem der Beispiele 1 bis 4, hergestellt werden.

**Patentansprüche für die Vertragsstaaten: BE, CH, DE, FR, GB, IT, LI, LU, NL, SE**

1. Neue 4-Acylresorcinäther der Formel

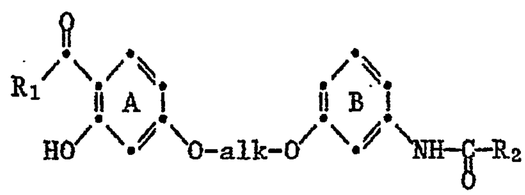

(I),

worin $R_1$ Niederalkyl bedeutet, alk einen gegebenenfalls durch Sauerstoff unterbrochenen Hydroxyniederalkylenrest darstellt und $R_2$ 5-Tetrazolyl bedeutet, wobei der Ring A zusätzlich durch Niederalkyl, Niederalkenyl, Niederalkinyl, Niederalkoxy und/oder Halogen und der Ring B zusätzlich durch Niederalkyl, Niederalkanoyl, Niederalkoxy, Niederalkoxycarbonyl, Carboxy, Cyano, Carbamyl, Halogen und/oder Trifluormethyl substituiert sein kann und wobei Halogen eine Atomnummer bis und mit 53 aufweisen kann und "niedere" organische Gruppen bis und mit 7 C-Atome besitzen können, und ihre Salze.

2. Verbindungen gemäß Anspruch 1 der Formel I, worin $R_1$ Niederalkyl bedeutet, alk für Hydroxyniederalkylen, Hydroxy(oxa)niederalkylen oder Hydroxy(dioxa)niederalkylen, worin die Hydroxygruppe in höherer als der $\alpha$- und in niedrigerer als der $\omega$-Stellung zu den freien Valenzen sowie in höherer als der $\alpha$-Stellung zu(m) gegebenenfalls vorhandenen, in höherer als der $\beta$- und niedrigerer als der $(\omega-1)$-Stellung zu den freien Valenzen befindlichen, Oxaglied(ern) gebunden ist, steht und $R_2$ 5-Tetrazolyl bedeutet, wobei der Ring A zusätzlich durch Niederalkyl, Niederalkenyl, Niederalkinyl, Niederalkoxy und/oder Halogen und der Ring B zusätzlich durch Niederalkyl, Niederalkanoyl, Niederalkoxycarbonyl, Carbamyl, Carboxy, Cyano, Halogen und/oder Trifluormethyl substituiert sein kann und wobei Halogen eine Atomnummer bis und mit 53 aufweisen kann und "niedere" organische Gruppen bis und mit 7 C-Atome besitzen können, und ihre Salze.

3. Verbindungen gemäß Anspruch 1 der Formel

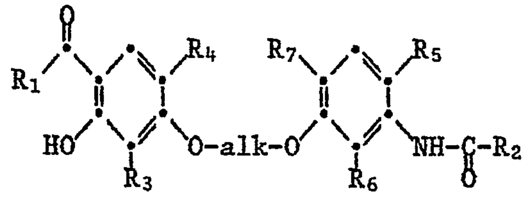

(Ia),

worin $R_1$ für Niederalkyl mit bis und mit 4 C-Atomen steht, $R_2$ 5-Tetrazolyl bedeutet, $R_3$ Niederalkyl mit bis und mit 4 C-Atomen darstellt, $R_4$ für Wasserstoff, Niederalkyl mit bis und mit 4 C-Atomen, Niederalkoxy mit bis und mit 4 C-Atomen oder Halogen der Atomnummer bis und mit 53 steht, $R_5$ Wasserstoff, Niederalkyl mit bis und mit 4 C-Atomen, Halogen der Atomnummer bis und mit 53 oder Trifluormethyl steht, $R_6$ Wasserstoff, Niederalkyl mit bis und mit 4 C-Atomen, Niederalkoxy mit bis und mit 4 C-Atomen, Cyano oder Halogen der Atomnummer bis und mit 53 darstellt, $R_7$ Wasserstoff, Niederalkyl mit bis und

mit 4 C-Atomen, Halogen der Atomnummer bis und mit 53, Niederalkoxycarbonyl mit bis und mit 5 C-Atomen, Carboxy, Cyano oder Niederalkanoyl mit bis und mit 7 C-Atomen bedeutet und alk für 1,3-(2-Hydroxy)propylen steht, und ihre Salze.

4. Verbindungen gemäß Anspruch 1 der Formel Ia, worin $R_1$ Niederalkyl mit bis und mit 4 C-Atomen bedeutet, $R_3$ geradkettiges Niederalkyl mit bis und mit 4 C-Atomen bedeutet, $R_2$ 5-Tetrazolyl drstellt, $R_4$ für Wasserstoff steht, $R_5$ Niederalkyl mit bis und mit 4 C-Atomen bedeutet, $R_6$ Wasserstoff darstellt, $R_7$ für Halogen mit einer Atomnummer von 17 bis und mit 53 oder Cyano steht und alk 1,3-(2-Hydroxy)propylen bedeutet, und ihre Salze mit Basen.

5. N-{3-[3-(4-Acetyl-3-hydroxy-2-n-propyl-phenoxy)-2-hydroxy-propyloxy]-4-brom-6-methyl-phenyl}-1H-tetrazol-5-carboxamid oder ein Salz davon.

6. N-{3-[3-(4-Acetyl-3-hydroxy-2-n-propyl-phenoxy)-3-hydroxy-propyloxy]-2-cyano-phenyl}-1H-tetrazol-5-carboxamid oder ein Salz davon.

7. N-{3-[3-(4-Acetyl-3-hydroxy-2-n-propyl-phenoxy)-2-hydroxy-propyloxy]-6-methyl-phenyl}-1H-tetrazol-5-carboxamid oder ein Salz davon.

8. N-{3-[3-(4-Acetyl-3-hydroxy-2-n-propyl-phenoxy)-2-hydroxy-propyloxy]-4-chlor-6-methyl-phenyl}-1H-tetrazol-5-carboxamid oder ein Salz davon.

9. N-{3-[3-(4-Acetyl-3-hydroxy-2-n-propyl-phenoxy)-2-hydroxy-propyloxy]-4-fluor-6-methylphenyl}-1H-tetrazol-5-carboxamid oder ein Salz davon.

10. N-{3-[3-(4-Acetyl-3-hydroxy-2-n-propyl-phenoxy)-2-hydroxy-propyloxy]-4-cyano-6-methylphenyl}-1H-tetrazol-5-carboxamid oder ein Salz davon.

11. Eine Verbindung gemäß einem der Ansprüche 1 bis 10 in Form des Natrium-, Kalium-, Triäthanolammonium-, Diäthylammonium-, Diäthanolammonium- oder Tris(hydroxymethyl)methylammoniumsalzes.

12. Eine Verbindung gemäß einem der Ansprüche 1 bis 11 zur Anwendung in einem Verfahren zur therapeutischen Behandlung des menschlichen oder tierischen Körpers.

13. Eine Verbindung gemäß einem der Ansprüche 1 bis 11 zur Anwendung gemäß Anspruch 12 als Antiallergikum und/oder Antiasthmatikum.

14. Pharmazeutische Präparate enthaltend eine Verbindung gemäß einem der Ansprüche 1 bis 11 neben üblichen Hilfs- und Trägerstoffen.

15. Verwendung einer Verbindung gemäß einem der Ansprüche 1 bis 11 zur Herstellung antiallergischer Arzneimittel.

16. Verfahren zur Herstellung einer Verbindung der Formel I oder eines Salzes davon gemäß einem der Ansprüche 1 bis 11, dadurch gekennzeichnet, daß man

a) eine Verbindung der Formel

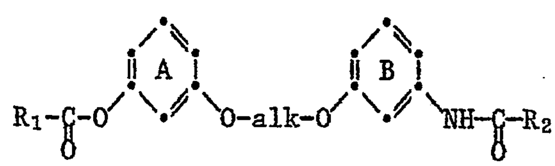

(II)

umlagert oder

b) eine Verbindung der Formel

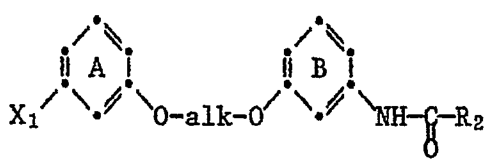

(III),

worin $X_1$ gegebenenfalls veräthertes Hydroxy bedeutet, mit einer Verbindung der Formel $R_1$–$X_2$ (IV), worin $X_2$ gegebenenfalls funktionell abgewandeltes Carboxy bedeutet, umsetzt oder

c) in einer Verbindung der Formel

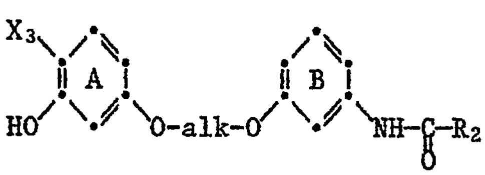

(V) ,

worin $X_3$ einen in die Gruppe der Formel $R_1$–C(=O)– überführbaren Rest bedeutet, $X_3$ in diese Gruppe überführt oder

d) in einer Verbindung der Formel

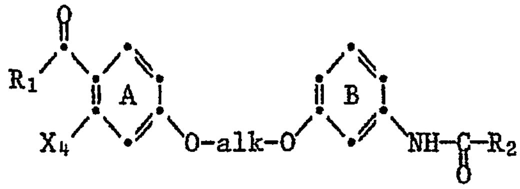 (VI),

worin $X_4$ einen in Hydroxy überführbaren Rest bedeutet, $X_4$ in Hydroxy überführt oder
e) Verbindungen der Formeln

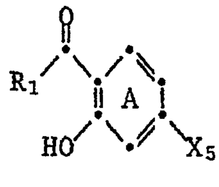 (VII) und 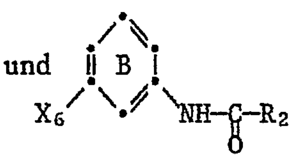 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest darstellt, wobei "niedere" organische Gruppen 3 bis und mit 7 C-Atome besitzen können, oder
f) in einer Verbindung der Formel

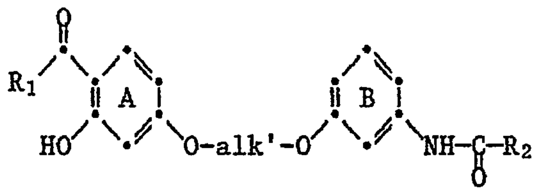 (IX),

worin alk' eine in einen Rest alk überführbare Gruppe bedeutet, alk' in einen Rest alk überführt oder
g) eine Verbindung der Formel

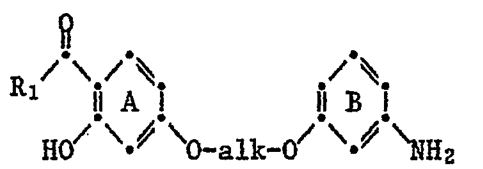 (X)

oder ein Salz davon mit einer Verbindung der Formel

$X_7–R_2$ (XI)

umsetzt, worin $X_7$ eine gegebenenfalls veresterte, amidierte, anhydridisierte oder in Salzform vorliegende Carboxygruppe bedeutet, oder
h) in einer Verbindung der Formel

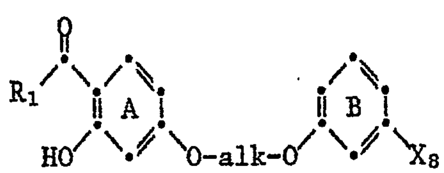 (XII),

worin $X_8$ einen in die gewünschte Gruppe der Formel $–NH–C(=0)–R_2$ überführbaren Rest bedeutet, $X_8$ in diese überführt und gewünschtenfalls eine verfahrensgemäß erhältliche Verbindung der Formel I in eine andere Verbindung der Formel I überführt und/oder eine verfahrensgemäß erhältliche freie Verbindung der Formel I in ein Salz oder ein verfahrensgemäß erhältliches Salz in die freie Verbindung der Formel I oder in ein anderes Salz umwandelt.

**Patentansprüche für den Vertragsstaat: AT**

1. Verfahren zur Herstellung neuer 4-Acylresorcinäther der Formel

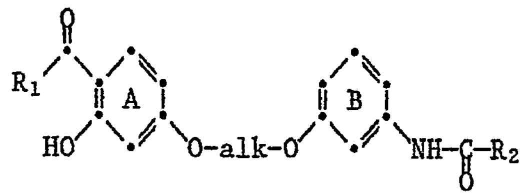

(I),

worin $R_1$ Niederalkyl bedeutet, alk einen gegebenenfalls durch Sauerstoff unterbrochenen Hydroxyniederalkylenrest darstellt und $R_2$ 5-Tetrazolyl bedeutet, wobei der Ring A zusätzlich durch Niederalkyl, Niederalkenyl, Niederalkinyl, Niederalkoxy, und/oder Halogen und der Ring B zusätzlich durch Niederalkyl, Niederalkanoyl, Niederalkoxy, Niederalkoxycarbonyl, Carboxy, Cyano, Carbamyl, Halogen und/oder Trifluormethyl substituiert sein kann und wobei Halogen eine Atomnummer bis und mit 53 aufweisen kann und "niedere" organische Gruppen bis und mit 7 C-Atome besitzen können, oder ihrer Salze, dadurch gekennzeichnet, daß man

a) eine Verbindung der Formel

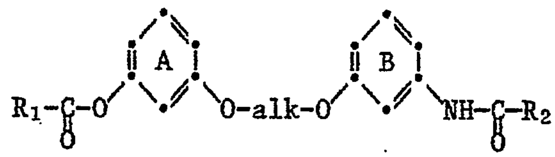

(II)

umlagert oder

b) eine Verbindung der Formel

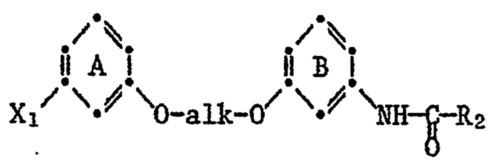

(III),

worin $X_1$ gegebenenfalls veräthertes Hydroxy bedeutet, mit einer Verbindung der Formel $R_1$–$X_2$ (IV), worin $X_2$ gegebenenfalls funktionell abgewandeltes Carboxy bedeutet, umsetzt oder

c) in einer Verbindung der Formel

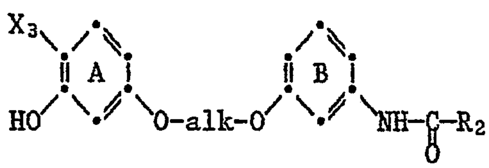

(V) ,

worin $X_3$ einen in die Gruppe der Formel $R_1$–C(=O)– überführbaren Rest bedeutet, $X_3$ in diese Gruppe überführt oder

d) in einer Verbindung der Formel

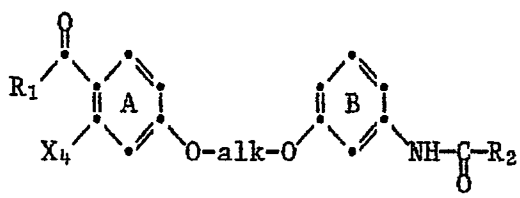

(VI),

worin $X_4$ einen in Hydroxy überführbaren Rest bedeutet, $X_4$ in Hydroxy überführt oder

e) Verbindungen der Formeln

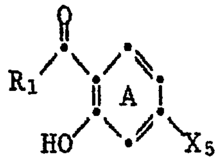 (VII) und 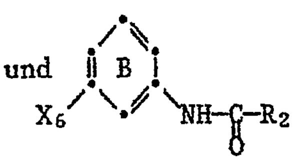 (VIII)

miteinander umsetzt, worin einer der Reste $X_5$ und $X_6$ gegebenenfalls in Salzform vorliegendes Hydroxy und der andere einen durch reaktionsfähiges verestertes Hydroxy substituierten, gegebenenfalls durch Sauerstoff unterbrochenen Hydroxyniederalkoxyrest oder einen gegebenenfalls durch Sauerstoff unterbrochenen Epoxyniederalkoxyrest darstellt, wobei "niedere" organische Gruppen 3 bis und mit 7 C-Atome besitzen können, oder

f) in einer Verbindung der Formel

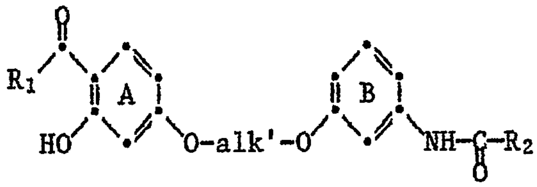 (IX),

worin alk' eine in einen Rest alk überführbare Gruppe bedeutet, alk' in einen Rest alk überführt oder

g) eine Verbindung der Formel

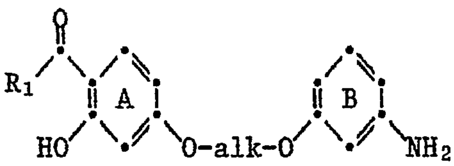 (X)

oder ein Salz davon mit einer Verbindung der Formel

$X_7–R_2$ (XI)

umsetzt, worin $X_7$ eine gegebenenfalls veresterte, amidierte, anhydridisierte oder in Salzform vorliegende Carboxygruppe bedeutet, oder

h) in einer Verbindung der Formel

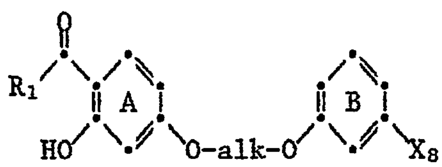 (XII),

worin $X_8$ einen in die gewünschte Gruppe der Formel $–NH–C(=O)–R_2$ überführbaren Rest bedeutet, $X_8$ in diese überführt und gewünschtenfalls eine verfahrensgemäß erhältliche Verbindung der Formel I in eine andere Verbindung der Formel I überführt und/oder eine verfahrensgemäß erhältliche freie Verbindung der Formel I in ein Salz oder ein verfahrensgemäß erhältliches Salz in die freie Verbindung der Formel I oder in ein anderes Salz umwandelt.

2. Verfahren gemäß Anspruch 1, dadurch gekennzeichnet, daß man Verbindungen der Formel I, worin $R_1$ Niederalkyl bedeutet, alk für Hydroxyniederalkylen, Hydroxy(oxa)niederalkylen oder Hydroxy(dioxa)niederalkylen, worin die Hydroxygruppe in höherer als der α- und in niedrigerer als der ω-Stellung zu den freien Valenzen sowie in höherer als der α-Stellung zu(m) gegebenenfalls vorhandenen, in höherer als der β- und in niedrigerer als der (ω-1)-Stellung zu den freien Valenzen befindlichen, Oxaglied(ern) gebunden ist, steht und $R_2$ 5-Tetrazolyl bedeutet, wobei der Ring A zusätzlich durch Niederalkyl, Niederalkenyl, Niederalkinyl, Niederalkoxy und/der Halogen und der Ring B durch Niederalkyl, Niederalkanoyl, Niederalkoxy, Niederalkoxycarbonyl, Carbamyl, Carboxy, Cyano, Halogen und/oder Trifluormethyl substituiert sein kann und wobei Halogen eine Atomnummer bis und mit 53 aufweisen kann und "niedere" organische Gruppen bis und mit 7 C-Atome besitzen können, oder ihre Salze herstellt.

3. Verfahren gemäß Anspruch 1, dadurch gekennzeichnet, daß man Verbindungen der Formel

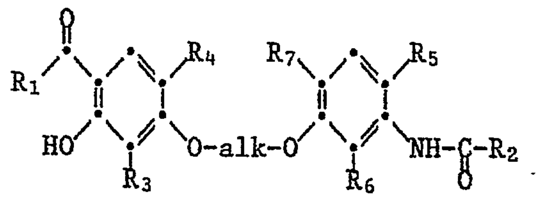

(Ia) ,

worin $R_1$ für Niederalkyl mit bis und mit 4 C-Atomen steht, $R_2$ 5-Tetrazolyl bedeutet, $R_3$ Niederalkyl mit bis und mit 4 C-Atomen darstellt, $R_4$ für Wasserstoff, Niederalkyl mit bis und mit 4 C-Atomen, Niederalkoxy mit bis und mit 4 C-Atomen oder Halogen der Atomnummer bis und mit 53 steht, $R_5$ für Wasserstoff, Niederalkyl mit bis und mit 4 C-Atomen, Halogen der Atomnummer bis und mit 53 oder Trifluormethyl steht, $R_6$ Wasserstoff, Niederalkyl mit bis und mit 4 C-Atomen, Niederalkoxy mit bis und mit 4 C-Atomen, Cyano oder Halogen der Atomnummer bis und mit 53 darstellt, $R_7$ Wasserstoff, Niederalkyl mit bis und mit 4 C-Atomen, Halogen der Atomnummer bis und mit 53, Niederalkoxycarbonyl mit bis und mit 5 C-Atomen, Carboxy, Cyano oder Niederalkanoyl mit bis und mit 7 C-Atomen bedeutet und alk für 1,3-(2-Hydroxy)propylen steht, oder ihre Salze herstellt.

4. Verfahren gemäß Anspruch 1, dadurch gekennzeichnet, daß man Verbindungen der Formel Ia, worin $R_1$ Niederalkyl mit bis und mit 4 C-Atomen bedeutet, $R_3$ geradkettiges Niederalkyl mit bis und mit 4 C-Atomen bedeutet, $R_2$ 5-Tetrazolyl darstellt, $R_4$ für Wasserstoff steht, $R_5$ Niederalkyl mit bis und mit 4 C-Atomen bedeutet, $R_6$ Wasserstoff darstellt, $R_7$ für Halogen mit einer Atomnummer von 17 bis und mit 53 oder Cyano steht und alk 1,3-(2-Hydroxy)propylen bedeutet, oder ihre Salze mit Basen herstellt.

5. Verfahren gemäß Anspruch 1, dadurch gekennzeichnet, daß man N-{3-[3-(4-Acetyl-3-hydroxy-2-n-propyl-phenoxy)-2-hydroxy-propyloxy]-4-brom-6-methyl-phenyl}-1H-tetrazol-5-carboxamid oder ein Salz davon herstellt.

6. Verfahren gemäß Anspruch 1, dadurch gekennzeichnet, daß man N-{3-[3-(4-Acetyl-3-hydroxy-2-n-propyl-phenoxy)-2-hydroxy-propyloxy]-2-cyano-phenyl}-1H-tetrazol-5-carboxamid oder ein Salz davon herstellt.

7. Verfahren gemäß Anspruch 1, dadurch gekennzeichnet, daß man N-{3-[3-(4-Acetyl-3-hydroxy-2-n-propyl-phenoxy)-2-hydroxy-propyloxy]-6-methyl-phenyl}-1H-tetrazol-5-carboxamid oder ein Salz davon herstellt.

8. Verfahren gemäß Anspruch 1, dadurch gekennzeichnet, daß man N-{3-[3-(4-Acetyl-3-hydroxy-2-n-propyl-phenoxy)-2-hydroxy-propyloxy]-4-chlor-6-methyl-phenyl}-1H-tetrazol-5-carboxamid oder ein Salz davon herstellt.

9. Verfahren gemäß Anspruch 1, dadurch gekennzeichnet, daß man N-{3-[3-(4-Acetyl-3-hydroxy-2-n-propyl-phenoxy)-2-hydroxy-propyloxy]-4-fluor-6-methylphenyl}-1H-tetrazol-5-carboxamid oder ein Salz davon herstellt.

10. Verfahren gemäß Anspruch 1, dadurch gekennzeichnet, daß man N-{3-[3-(4-Acetyl-3-hydroxy-2-n-propyl-phenoxy)-2-hydroxy-propyloxy]-4-cyano-6-methyl-phenyl}-1H-tetrazol-5-carboxamid oder ein Salz davon herstellt.

11. Verfahren gemäß Anspruch 1, dadurch gekennzeichnet, daß man eine Verbindung, erhältlich gemäß einem der Ansprüche 1 bis 10, in Form des Natrium-, Kalium-, Triäthanolammonium-, Diäthylammonium-, Diäthanolammonium- oder Tris(hydroxymethyl)methylammoniumsalzes herstellt.

12. Verfahren zur Herstellung pharmazeutischer Präparate, dadurch gekennzeichnet, daß man eine Verbindung, erhältlich gemäß einem der Ansprüche 1 bis 11, mit üblichen Hilfs- und Trägerstoffen vermischt.

**Claims for the Contracting States: BE, CH, DE, FR, GB, IT, LI, LU, NL, SE**

1. A novel 4-acylresorcinol ether of the formula

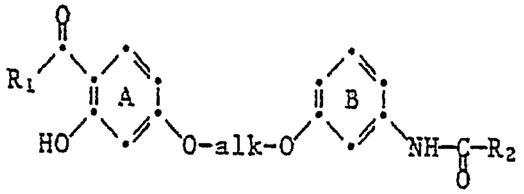

(I)

in which $R_1$ is lower alkyl, alk is a hydroxy-lower alkylene radical which can be interrupted by oxygen and $R_2$ is 5-tetrazolyl, it being possible for the ring A to be additionally substituted by lower alkyl, lower alkenyl, lower alkynyl, lower alkoxy and/or halogen and for the ring B to be additionally substituted by lower alkyl, lower alkanoyl, lower alkoxy, lower alkoxycarbonyl, carboxyl, cyano, carbamyl, halogen and/or trifluoromethyl, and it being possible for halogen to have an atomic number not greater than 53 and for "lower" organic groups to have not more than 7 C atoms, or a salt thereof.

2. A compound according to claim 1 of the formula I, in which $R_1$ is lower alkyl, alk is hydroxy-lower alkylene, hydroxy-(oxa)-lower alkylene or hydroxy-(dioxa)-lower alkylene in which the hydroxyl group is attached in a position higher than the α-position and lower than the ω-position in relation to the free valences and in a position higher than the α-position in relation to (the) oxa member(s) which can be present and are located in a position higher than the β-position and lower than the (ω-1)-position in relation to the free valences, and $R_2$ is 5-tetrazolyl, it being possible for the ring A to be additionally substituted by lower alkyl, lower alkenyl, lower alkoxy and/or halogen and for the ring B to be additionally substituted by lower alkyl, lower alkanoyl, lower alkoxy, lower alkoxycarbonyl, carbamyl, carboxyl, cyano, halogen and/or trifluoromethyl, and it being possible for halogen to have an atomic number not greater than 53 and for "lower" organic groups to have not more than 7 C atoms, or a salt thereof.

3. A compound according to claim 1 of the formula

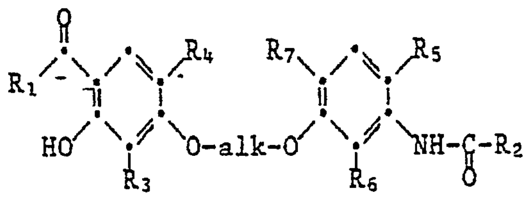

(Ia)

in which $R_1$ is lower alkyl having not more than 4 C atoms, $R_2$ is 5-tetrazolyl, $R_3$ is lower alkyl having not more than 4 C atoms, $R_4$ is hydrogen, lower alkyl having not more than 4 C atoms, lower alkoxy having not more than 4 C atoms or halogen of atomic number not greater than 53, $R_5$ is hydrogen, lower alkyl having not more than 4 C atoms, halogen of atomic number not greater than 53 or trifluoromethyl, $R_6$ is hydrogen, lower alkyl having not more than 4 C atoms, lower alkoxy having not more than 4 C atoms, cyano or halogen of atomic number not greater than 53 and $R_7$ is hydrogen, lower alkyl having not more than 4 C atoms, halogen of atomic number not greater than 53, lower alkoxycarbonyl having not more than 7 C atoms and alk is 1,3-(2-hydroxy)-propylene, or a salt thereof.

4. A compound according to claim 1, of the formula Ia in which $R_1$ is lower alkyl having not more than 4 C atoms, $R_3$ is linear lower alkyl having not more than 4 C atoms, $R_2$ is 5-tetrazolyl, $R_4$ is hydrogen, $R_5$ is lower alkyl having not more than 4 C atoms, $R_6$ is hydrogen, $R_7$ is halogen having an atomic number of from 17 to not greater than 53 or cyano and alk is 1,3-(2-hydroxy)-propylene or a salt thereof with bases.

5. N-{3-[3-(4-acetyl-3-hydroxy-2-n-propylphenoxy)-2-hydroxypropoxy]-4-bromo-6-methylphenyl}-1H-tetrazole-5-carboxamide or a salt thereof.

6. N-{3-[3-(4-acetyl-3-hydroxy-2-n-propylphenoxy)-3-hydroxypropoxy]-2-cyanophenyl}-1H-tetrazole-5-carboxamide or a salt thereof.

7. N-{3-[3-(4-acetyl-3-hydroxy-2-n-propylphenoxy)-2-hydroxypropoxy]-6-methylphenyl}-1H-tetrazole-5-carboxamide or a salt thereof.

8. N-{3-[3-(4-acetyl-3-hydroxy-2-n-propylphenoxy)-2-hydroxypropoxy]-4-chloro-6-methylphenyl}-1H-tetrazole-5-carboxamide or a salt thereof.

9. N-{3-[3-(4-acetyl-3-hydroxy-2-n-propylphenoxy)-2-hydroxypropoxy]-4-fluoro-6-methylphenyl}-1H-tetrazole-5-carboxamide or a salt thereof.

10. N-{3-[3-(4-acetyl-3-hydroxy-2-n-propylphenoxy)-2-hydroxypropoxy]-4-cyano-6-methylphenyl}-1H-tetrazole-5-carboxamide or a salt thereof.

11. A compound according to any of claims 1 to 10 in the form of the sodium, potassium, triethanolammonium, diethylammonium, diethanolammonium or tris-(hydroxymethyl)-methylammonium salt.

12. A compound according to any one of claims 1 to 11 for use in a process for the therapeutic treatment of the human or animal body.

13. A compound according to any one of claims 1 to 11 for use in accordance with claim 12 as an antiallergic agent and/or antiasthmatic agent.

14. A pharmaceutical formulation containing a compound according to any one of claims 1 to 11 together with customary adjuncts and excipients.

15. The use of a compound according to any one of claims 1 to 11 for the preparation of antiallergic medicaments.

16. A process for the preparation of a compound of the formula I or a salt thereof according to any one of claims 1 to 11, which comprises

a) subjecting a compound of the formula

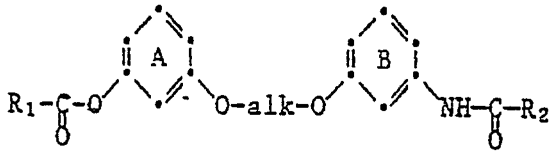

(II)

to a rearrangement or b) reacting a compound of the formula

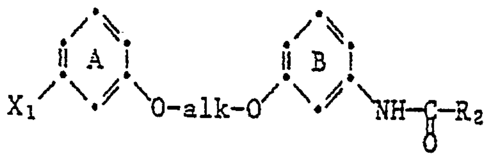

(III)

in which $X_1$ is free or etherified hydroxyl with a compound of the formula $R_1–X_2$ (IV) in which $X_2$ is free or functionally modified carboxyl, or c) in a compound of the formula

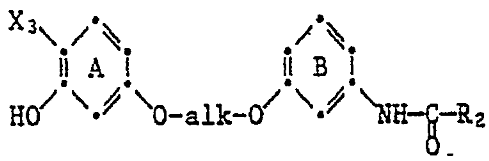

(V)

in which $X_3$ is a radical which can be converted into the group of the formula $R_1–C(=O)–$, converting $X_3$ into this group or d) in a compound of the formula

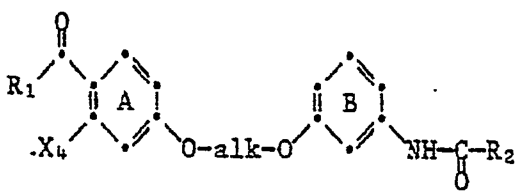

(VI)

in which $X_4$ is a radical which can be converted into hydroxyl, converting $X_4$ into hydroxyl, or e) reacting with one another compounds of the formulae

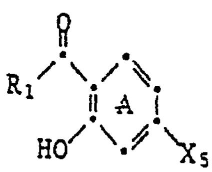

(VII) and

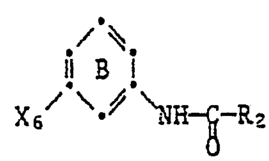

(VIII)

in which one of the radicals $X_5$ and $X_6$ is free hydroxyl or hydroxyl in the form of a salt and the other is a hydroxy-lower alkoxy radical which is substituted by reactive hydroxyl and can be interrupted by oxygen or is an epoxy-lower alkoxy radical which can be interrupted by oxygen, it being possible for "lower" organic groups to have from 3 to not more than 7 C atoms, or f) in a compound of the formula

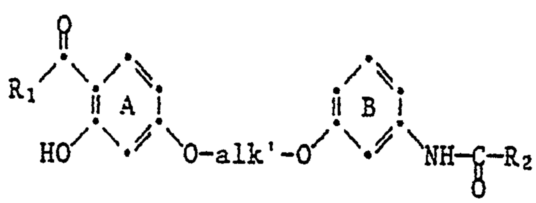

(IX)

in which alk' is a group which can be converted into a radical alk, converting alk' into a radical alk, or g) reacting a compound of the formula

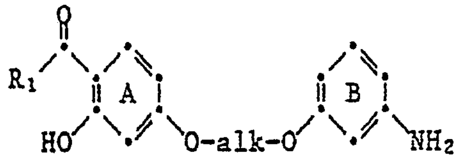

(X)

or a salt thereof, with a compound of the formula

$X_7$–$R_2$ (XI)

in which $X_7$ is a free carboxyl group or an esterified or amidised carboxyl group or a carboxyl group in the form of an anhydride or salt, or h) in a compound of the formula

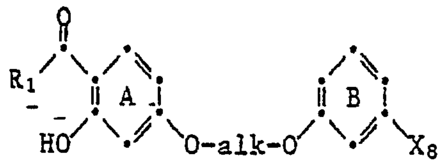

(XII)

in which $X_8$ is a radical which can be converted into the desired group of the formula –NH–C–(=O)–$R_2$, converting $X_8$ into this group and, if desired, converting a compound of the formula I obtainable in accordance with the process into another compound of the formula I and/or converting a free compound of the formula I obtainable in accordance with the process into a salt or converting a salt obtainable in accordance with the process into the free compound of the formula I or into another salt.

**Claims for the Contracting State: AT**

1. A process for the preparation of a novel 4-acylresorcinol ether of the formula

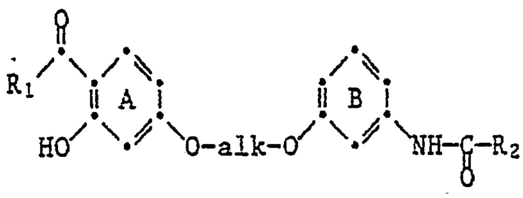

(I)

in which $R_1$ is lower alkyl, alk is a hydroxy-lower alkylene radical which can be interrupted by oxygen and $R_2$ is 5-tetrazolyl, it being possible for the ring A to be additionally substituted by lower alkyl, lower alkenyl, lower alkynyl, lower alkoxy and/or halogen and for the ring B to be additionally substituted by lower alkyl, lower alkanoyl, lower alkoxy, lower alkoxycarbonyl, carboxyl, cyano, carbamyl, halogen and/or trifluoromethyl, and it being possible for halogen to have an atomic number not greater than 53 and for "lower" organic groups to have not more than 7 C atoms, or a salt thereof, which comprises a) subjecting a compound of the formula

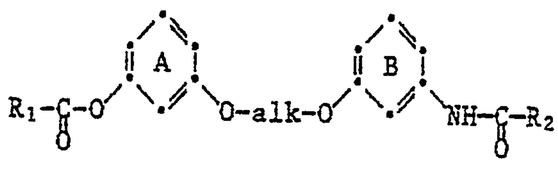

(II)

to a rearrangement or b) reacting a compound of the formula

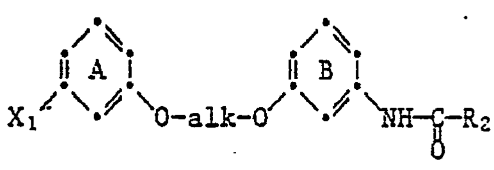

(III).

in which $X_1$ is free or etherified hydroxyl with a compound of the formula $R_1$–$X_2$ (IV) in which $X_2$ is free or functionally modified carboxyl, or c) in a compound of the formula

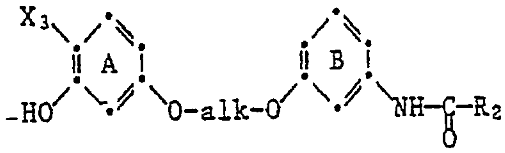

(V)

in which $X_3$ is a radical which can be converted into the group of the formula $R_1–C(=O)–$, converting $X_3$ into this group or d) in a compound of the formula

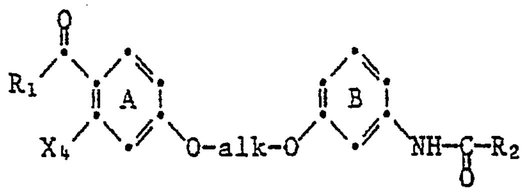

(VI)

in which $X_4$ is a radical which can be converted into hydroxyl, converting $X_4$ into hydroxyl, or e) reacting with one another compounds of the formulae

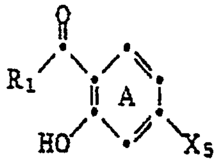

(VII) and

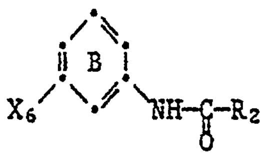

(VIII)

in which one of the radicals $X_5$ and $X_6$ is free hydroxyl or hydroxyl in the form of a salt and the other is a hydroxy-lower alkoxy radical which is substituted by reactive esterified hydroxyl and can be interrupted by oxygen or is an epoxy-lower alkoxy radical which can be interrupted by oxygen, it being possible for "lower" organic groups to have from 3 to not more than 7 C atoms, or f) in a compound of the formula

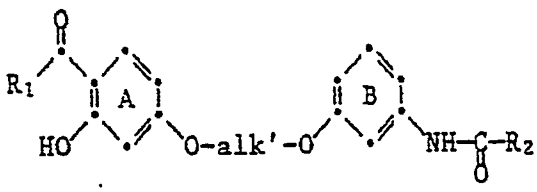

(IX)

in which alk' is a group which can be converted into a radical alk, converting alk' into a radical alk, or g) reacting a compound of the formula

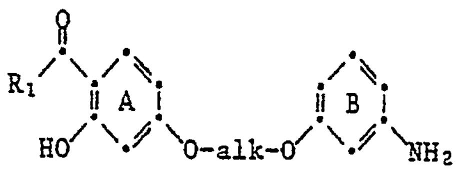

(X)

or a salt thereof, with a compound of the formula

$$X_7–R_2 \quad (XI)$$

in which $X_7$ is a free carboxyl group or an esterified or amidised carboxyl group or a carboxyl group in the form of an anhydride or salt, or h) in a compound of the formula

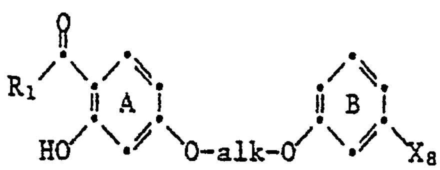

(XII)

in which $X_8$ is a radical which can be converted into the desired group of the formula $-NH-C-(=O)-R_2$, converting $X_8$ into this group and, if desired, converting a compound of the formula I obtainable in accordance with the process into another compound of the formula I and/or converting a free compound of the formula I obtainable in accordance with the process into a salt or converting a salt obtainable in accordance with the process into the free compound of the formula I or into another salt.

2. A process according to claim 1, which comprises preparing a compound of the formula I, in which $R_1$ is lower alkyl, alk is hydroxy-lower alkylene, hydroxy-(oxa)-lower alkylene or hydroxy-(dioxa)-lower alkylene in which the hydroxyl group is attached in a position higher than the $\alpha$-position and lower than the $\omega$-position in relation to the free valences and in a position higher than the $\alpha$-position in relation to (the) oxa member(s) which can be present and are located in a position higher than the $\beta$-position and lower than the ($\omega$-1)-position in relation to the free valences, and $R_2$ is 5-tetrazolyl, it being possible for the ring A to be additionally substituted by lower alkyl, lower alkenyl, lower alkynyl, lower alkoxy and/or halogen and for the ring B to be additionally substituted by lower alkyl, lower alkanoyl, lower alkoxy, lower alkoxycarbonyl, carbamyl, carboxyl, cyano, halogen and/or trifluoromethyl, and it being possible for halogen to have an atomic number not greater than 53 and for "lower" organic groups to have not more than 7 C atoms, or a salt thereof.

3. A process according to claim 1, which comprises preparing a compound of the formula

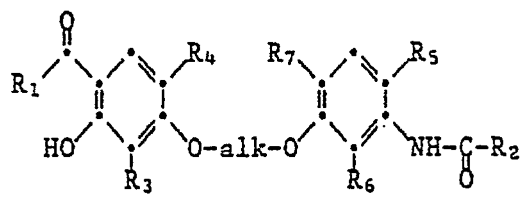

(Ia)

in which $R_1$ is lower alkyl having not more than 4 C atoms, $R_2$ is 5-tetrazolyl, $R_3$ is lower alkyl having not more than 4 C atoms, $R_4$ is hydrogen, lower alkyl having not more than 4 C atoms, lower alkoxy having not more than 4 C atoms or halogen of atomic number not greater than 53, $R_5$ is hydrogen, lower alkyl having not more than 4 C atoms, halogen of atomic number not greater than 53 or trifluoromethyl, $R_6$ is hydrogen, lower alkyl having not more than 4 C atoms, lower alkoxy having not more than 4 C atoms, cyano or halogen of atomic number not greater than 53 and $R_7$ is hydrogen, lower alkyl having not more than 4 C atoms, halogen of atomic number not greater than 53, lower alkoxycarbonyl having not more than 5 C atoms and alk is 1,3-(2-hydroxy)-propylene, or a salt thereof.

4. A process according to claim 1 which comprises preparing a compound of the formula Ia in which $R_1$ is lower alkyl having not more than 4 C atoms, $R_3$ is linear lower alkyl having not more than 4 C atoms, $R_2$ is 5-tetrazolyl, $R_4$ is hydrogen, $R_5$ is lower alkyl having not more than 4 C atoms, $R_6$ is hydrogen, $R_7$ is halogen having an atomic number of from 17 to not greater than 53 or cyano and alk is 1,3-(2-hydroxy)-propylene or a salt thereof with bases.

5. A process according to claim 1, which comprises preparing N-{3-[3-(4-acetyl-3-hydroxy-2-n-propylphenoxy)-2-hydroxypropoxy]-4-bromo-6-methylphenyl}-1H-tetrazole-5-carboxamide or a salt thereof.

6. A process according to claim 1, which comprises preparing N-{3-[3-(4-acetyl-3-hydroxy-2-n-propylphenoxy)-2-hydroxypropoxy]-2-cyanophenyl}-1H-tetrazole-5-carboxamide or a salt thereof.

7. A process according to claim 1, which comprises preparing N-{3-[3-(4-acetyl-3-hydroxy-2-n-propylphenoxy)-2-hydroxypropoxy]-6-methylphenyl}-1H-tetrazole-5-carboxamide or a salt thereof.

8. A process according to claim 1, which comprises preparing N-{3-[3-(4-acetyl-3-hydroxy-2-n-propylphenoxy)-2-hydroxypropoxy]-4-chloro-6-methylphenyl}-1H-tetrazole-5-carboxamide or a salt thereof.

9. A process according to claim 1, which comprises preparing N-{3-[3-(4-acetyl-3-hydroxy-2-n-propylphenoxy)-2-hydroxypropoxy]-4-fluoro-6-methylphenyl}-1H-tetrazole-5-carboxamide or a salt thereof.

10. A process according to claim 1, which comprises preparing N-{3-[3-(4-acetyl-3-hydroxy-2-n-propylphenoxy)-2-hydroxypropoxy]-4-cyano-6-methylphenyl}-1H-tetrazole-5-carboxamide or a salt thereof.

11. A process according to claim 1, which comprises preparing a compound obtainable according to any one of claims 1 to 10 in the form of the sodium, potassium, triethanolammonium, diethylammonium, diethanolammonium or tris-(hydroxymethyl)-methylammonium.

12. A process for the preparation of a pharmaceutical which comprises mixing a compound obtainable according to any one of claims 1 to 11 with customary adjuncts and excipients.

**Revendications pour les Etats contractants: BE CH DE FR GB IT LI LU NL SE**

1. Nouveaux éthers de 4-acylrésorcinols de formule

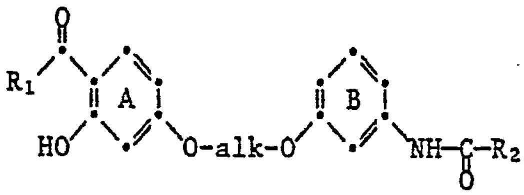

(I),

dans laquelle $R_1$ représente un groupe alkyle inférieur, alk représente un groupe hydroxyalkylène inférieur éventuellement interrompu par l'oxygène et $R_2$ un groupe 5-tétrazolyle, le cycle A pouvant en outre être substitué par des groupes alkyle inférieurs, alcényle inférieurs, alcynyle inférieurs, alcoxy inférieurs et/ou des halogènes et le cycle B par des groupes alkyle inférieurs, alcanoyle inférieurs, alcoxy inférieurs, (alcoxy inférieurs)-carbonyle, carboxy, cyano, carbamyle, des halogènes et/ou des groupes trifluorométhyle, les halogènes en question ayant un numéro atomique qui peut aller jusqu'à 53 inclus et des groupes organiques "inférieurs" pouvant contenir jusqu'à 7 atomes de carbones inclus, et leurs sels.

2. Composés selon la revendication 1, de formule I dans laquelle $R_1$ représente un gorupe alkyle inférieur, alk représente un groupe hydroxyalkylène inférieur, hydroxy(oxa)alkylène inférieur ou hydroxy(dioxa)alkylène inférieur dans lesquels le groupe hydroxy est en position supérieure à la position alpha et inférieure à la position oméga par rapport aux valences libres et en position supérieure à la position alpha par rapport aux chaînons oxa éventuellement présents, qui sont eux-mêmes en position supérieure à la position bêta et inférieure à la position (oméga-1) par rapport aux valences libres, et $R_2$ représente un groupe 5-tétrazolyle, le cycle A pouvant en outre être substitué par des groupes alkyle inférieurs, alcényle inférieurs, alcynyle inférieurs, alcoxy inférieurs et/ou des halogènes et le cycle B par des groupes alkyle inférieurs, alcanoyle inférieurs, alcoxy inférieurs, (alcoxy inférieurs)-carbonyle, carbamyle, carboxy, cyano, des halogènes et/ou des groupes trifluorométhyle, les halogènes en question ayant un numéro atomique qui peut aller jusqu'à 53 inclus et des groupes organiques "inférieurs" pouvant contenir jusqu'à 7 atomes de carbone inclus, et leurs sels.

3. Composés selon la revendication 1, de formule

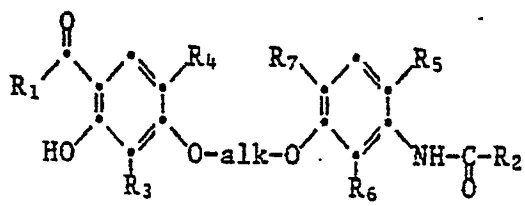

(Ia),

dans laquelle $R_1$ représente un groupe alkyle inférieur contenant jusqu'à 4 atomes de carbone inclus, $R_2$ représente un groupe 5-tétrazolyle, $R_3$ représente un groupe alkyle inférieur contenant jusqu'à 4 atomes de carbone inclus, $R_4$ représente l'hydrogène, un groupe alkyle inférieur contenant jusqu'à 4 atomes de carbone inclus, alcoxy inférieur contenant jusqu'à 4 atomes de carbone inclus ou un halogène de numéro atomique allant jusqu'à 53 inclus, $R_5$ représente l'hydrogène, un groupe alkyle inférieur contenant jusqu'à 4 atomes de carbone inclus, un halogène de numéro atomique allant jusqu'à 53 inclus ou un groupe trifluorométhyle, $R_6$ représente l'hydrogène, un groupe alkyle inférieur contenant jusqu'à 4 atomes de carbone inclus, alcoxy inférieur contenant jusqu'à 4 atomes de carbone inclus, cyano ou un halogène de numéro atomique allant jusqu'à 53 inclus, $R_7$ représente l'hydrogène, un groupe alkyle inférieur contenant jusqu'à 4 atomes de carbone inclus, un halogène de numéro atomique allant jusqu'à 53 inclus, un groupe (alcoxy inférieur)-carbonyle contenant jusqu'à 5 atomes de carbone inclus, carboxy, cyano ou alcanoyle inférieur contenant jusqu'à 7 atomes de carbone inclus et alk représente un groupe 1,3-(2-hydroxy)-propylène, et leurs sels.

4. Composés selon la revendication 1, de formule Ia, dans laquelle $R_1$ représente un groupe alkyle inférieur contenant jusqu'à 4 atomes de carbone inclus, $R_3$ représente un groupe alkyle inférieur à chaîne droite contenant jusqu'à 4 atomes de carbone inclus, $R_2$ représente un groupe 5-tétrazolyle, $R_4$ représente l'hydrogène, $R_5$ représente un groupe alkyle inférieur contenant jusqu'à 4 atomes de carbone inclus, $R_6$ représente l'hydrogène, $R_7$ représente un halogène de numéro atomique 17 à 53 inclus ou un groupe cyano et alk représente un groupe 1,3-(2-hydroxy)-propylène, et leurs sels de bases.

5. Le N-{3-[3-(4-acétyl-3-hydroxy-2-n-propyl-phénoxy)-2-hydroxy-propyloxy]-2-hydroxy-propyloxy]-4-bromo-6-méthyl-phényl}-1H-tétrazole-5-carboxamide ou un sel de ce composé.

6. Le N-{3-[3-(4-acétyl-3-hydroxy-2-n-propyl-phénoxy)-2-hydroxy-propyloxy]-2-cyano-phényl}-1H-tétrazole-5-carboxamide ou un sel de ce composé.

7. Le N-{3-[3-(4-acétyl-3-hydroxy-2-n-propyl-phénoxy)-2-hydroxy-propyloxy]-6-méthyl-phényl}-1H-tétrazole-5-carboxamide ou un sel de ce composé.

8. Le N-{3-[3-(4-acétyl-3-hydroxy-2-n-propyl-phénoxy)-2-hydroxy-propyloxy]-4-chloro-6-méthyl-phényl}-1H-tétrazole-5-carboxamide ou un sel de ce composé.

9. Le N-{3-[3-(4-acétyl-3-hydroxy-2-n-propyl-phénoxy)-2-hydroxy-propyloxy]-4-fluoro-6-méthyl-phényl}-1H-tétrazole-5-carboxamide ou un sel de ce composé.

10. Le N-{3-[3-(4-acétyl-3-hydroxy-2-n-propyl-phénoxy)-2-hydroxy-propyloxy]-4-cyano-6-méthyl-phényl}-1H-tétrazole-5-carboxamide ou un sel de ce composé.

11. Un composé selon l'une des revendications 1 à 10, à l'état de sel de sodium, de potassium, de triéthanolammonium, de diéthylammonium, de diéthanolammonium ou de tris-(hydroxyméthyl)-méthylammonium.

12. Un composé selon l'une des revendications 1 à 11, pour l'utilisation dans un procédé pour le traitement thérapeutique de l'organisme humain ou animal.

13. Un composé selon l'une des revendications 1 à 11, pour l'utilisation selon la revendication 12 en tant qu'antiallergique et/ou antiasthmatique.

14. Compositions pharmaceutiques contenant un composé selon l'une des revendications 1 à 11 avec des produits auxiliaires et véhicules usuels.

15. Utilisation d'un composé selon l'une des revendications 1 à 11 pour la préparation de médicaments antiallergiques.

16. Procédé de préparation d'un composé de formule I ou d'un sel d'un tel composé selon l'une des revendications 1 à 11, caractérisé en ce que:

a) on soumet un composé de formule

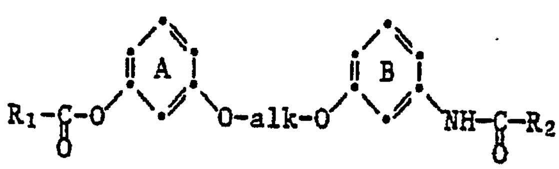

(II)

à transposition, ou bien

b) on fait réagir un composé de formule

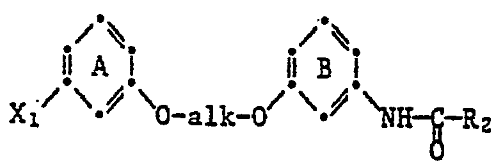

(III),

dans laquelle $X_1$ représente un groupe hydroxy éventuellement éthérifié, avec un composé de formule $R_1–X_2$ (IV) dans laquelle $X_2$ représente un groupe carboxy ayant éventuellement subi une modification fonctionnelle, ou bien

c) dans un composé de formule

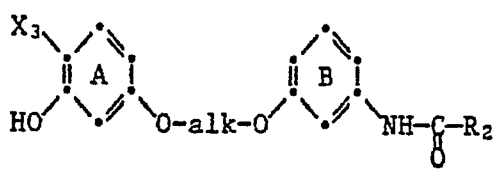

(V) ,

dans laquelle $X_3$ représente un substituant convertible en le groupe de formule $R_1–C(=O)–$, on convertit $X_3$ en ce groupe, ou bien

d) dans un composé de formule

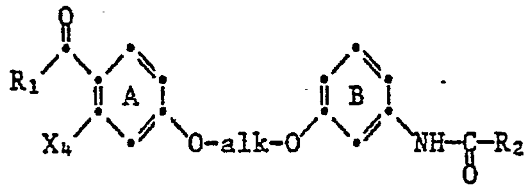

(VI),

dans laquelle $X_4$ représente un substituant convertible en groupe hydroxy, on convertit $X_4$ en groupe hydroxy, ou bien

e) on fait réagir entre eux les composés de formules

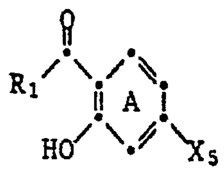

(VII) et

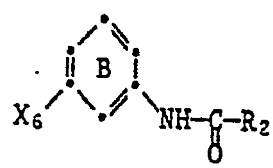

(VIII)

dans lesquelles l'un des symboles $X_5$ et $X_6$ représente un groupe hydroxy éventuellement à l'état de sel et l'autre un groupe hydroxyalcoxy inférieur substitué par un groupe hydroxy à l'état d'ester réactif et éventuellement interrompu par l'oxygène ou un groupe époxyalcoxy inférieur éventuellement interrompu par l'oxygène, les groupes organiques "inférieurs" pouvant contenir de 3 à 7 atomes de carbone inclus,

f) dans un composé de formule

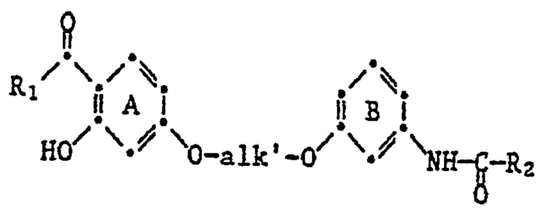

(IX),

dans laquelle alk' représente un groupe convertible en groupe alk, on convertit alk' en un group alk, ou bien

g) on fait réagir un composé de formule

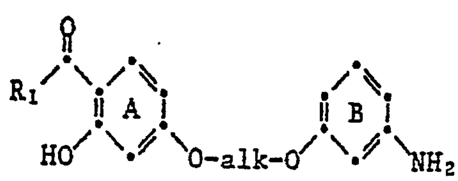

(X)

ou un sel d'un tel composé, avec un composé de formule

$X_7$–$R_2$ (XI)

dans laquelle $X_7$ représente un groupe carboxy éventuellement estérifié, amidé, anhydrisé ou à l'état de sel, ou bien

h) dans un composé de formule

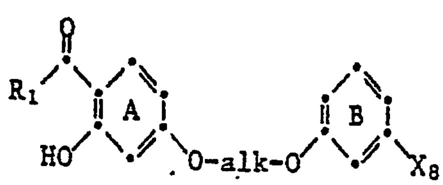

(XII),

dans laquelle $X_8$ représente un substituant convertible en le groupe recherché de formule –NH–C(=O)–$R_2$, on convertit $X_8$ en ce groupe, et si on le désire, on convertit un composé de formule I ainsi obtenu en un autre composé de formule I et/ou un composé libre de formule I ainsi obtenu en un sel ou un sel ainsi obtenu en le composé libre de formule I ou en un autre sel.

**Revendications pour l'Etat contractant: AT**

1. Procédé de préparation de nouveaux éthers de 4-acylrésorcinols de formule

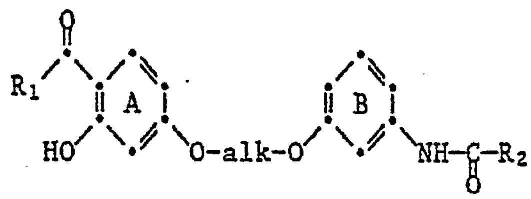

(I),

dans laquelle $R_1$ représente un groupe alkyle inférieur, alk représente un groupe hydroxyalkylène éventuellement interrompu par l'oxygène et $R_2$ représente un groupe 5-tétrazolyle, le cycle A pouvant en outre être substitué par des groupes alkyle inférieurs, alcényle inférieurs, alcynyle inférieurs, alcoxy inférieurs et/ou des halogènes et le cycle B par les groupes alkyle inférieurs, alcanoyle inférieurs, alcoxy inférieurs, (alcoxy inférieur)-carbonyle, carboxy, cyano, carbamyle, des halogènes et/ou des groupes trifluorométhyle, les halogènes ayant un numéro atomique qui peut aller jusqu'à 53 inclus et les groupes organiques "inférieurs" pouvant contenir jusqu'à 7 atomes de carbone inclus, ou de leurs sels, caractérisé en ce que

a) on soumet un composé de formule

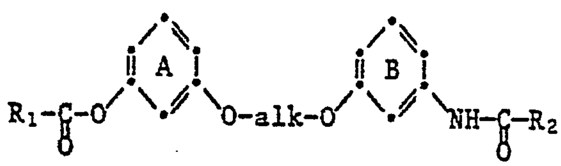

(II)

à transposition, ou bien

b) on fait réagir un composé de formule

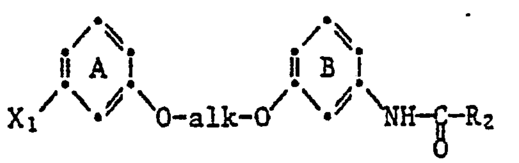

(III),

dans laquelle $X_1$ représente un groupe hydroxy éventuellement éthérifié, avec un composé de formule $R_1-X_2$ (IV) dans laquelle $X_2$ représente un groupe carboxy ayant subi éventuellement une modification fonctionnelle, ou bien

c) dans un composé de formule

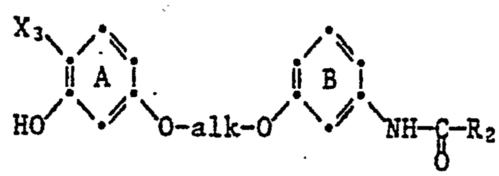

(V) ,

dans laquelle $X_3$ représente un substituant convertible en le groupe de formule $R_1-C(=O)-$, on convertit $X_3$ en ce groupe, ou bien

d) dans un composé de formule

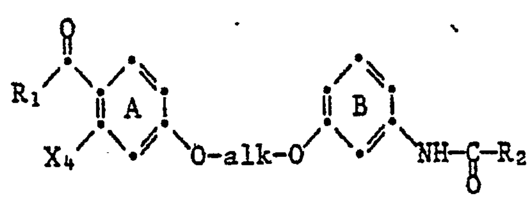

(VI),

dans laquelle $X_4$ représente un substituant convertible en groupe hydroxy, on convertit $X_4$ en groupe hydroxy,

e) on fait réagir entre eux les composés de formules

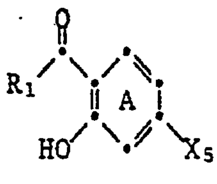

(VII) et

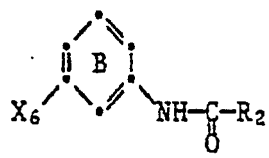

(VIII)

dans lesquelles l'un des symboles $X_5$ et $X_6$ représente un groupe hydroxy éventuellement à l'état de sel et l'autre un groupe hydroxyalcoxy inférieur substitué par un groupe hydroxy à l'état d'ester réactif et éventuellement interrompu par l'oxygène, les groupes organiques "inférieurs" pouvant contenir de 3 à 7 atomes de carbone inclus, ou bien

f) dans un composé de formule

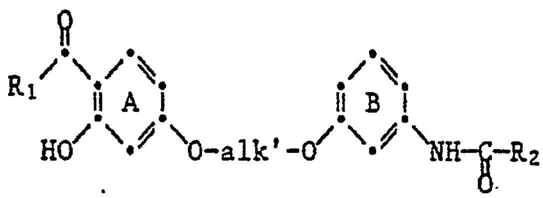

(IX),

dans laquelle alk' représente un groupe convertible en le groupe alk, on convertit alk' en le groupe alk, ou bien

g) on fait réagir un composé de formule

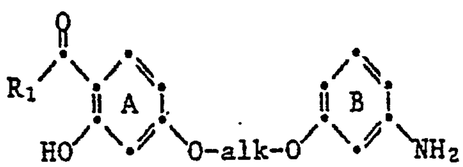

(X)

ou un sel d'un tel composé, avec un composé de formule

$X_7-R_2$ (XI)

dans laquelle $X_7$ représente un groupe carboxy éventuellement estérifié, amidé, anhydrisé ou à l'état de sel, ou bien

h) dans un composé de formule

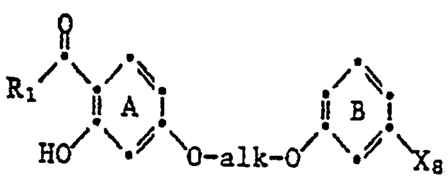

(XII),

dans laquelle $X_8$ représente un substituant convertible en le groupe recherché de formule $-NH-C(=O)-R_2$, on convertit $X_8$ en ce groupe et si on le désire, on convertit un composé de formule I ainsi obtenu en un autre composé de formule I et/ou un composé libre de formule I ainsi obtenu en un sel ou un sel ainsi obtenu en le composé libre de formule I ou en un autre sel.

2. Procédé selon la revendication 1, caractérisé en ce que l'on prépare des composés de formule I dans laquelle $R_1$ représente un groupe alkyle inférieur, alk représente un groupe hydroxyalkylène inférieur, hydroxy(oxa)alkylène inférieur ou hydroxy(dioxa)alkylène dans lesquels le groupe hydroxy est en position supérieure à la position alpha et inférieure à la position oméga par rapport aux valences libres et en position supérieure à la position alpha par rapport aux chaînons éventuels oxa, eux-mêmes en position supérieure à la position bêta et inférieure à la position (oméga-1) par rapport aux valences libres, et $R_2$ représente un groupe 5-tétrazolyle, le cycle A pouvant en outre être substitué par des groupes alkyle inférieurs, alcényle inférieurs, alcynyle inférieurs, alcoxy inférieurs et/ou des halogènes et le cycle B par des groupes alkyle inférieurs, alcanoyle inférieurs, alcoxy inférieurs, (alcoxy inférieurs)-carbonyle, carbamyle, carboxy, cyano, des halogènes et/ou des groupes trifluorométhyle, les halogènes en question ayant un numéro atomique allant jusqu'à 53 inclus et les groupes organiques "inférieurs" pouvant contenir jusqu'à 7 atomes de carbone, ou leurs sels.

3. Procédé selon la revendication 1, caractérisé en ce que l'on prépare des composés de formule

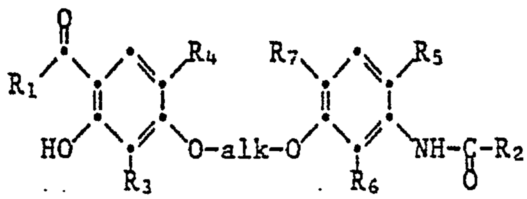

(Ia) ,

dans laquelle $R_1$ représente un groupe alkyle inférieur contenant jusqu'à 4 atomes de carbone inclus, $R_2$ représente un groupe 5-tétrazolyle, $R_3$ représente un groupe alkyle inférieur contenant jusqu'à 4 atomes de carbone inclus, $R_4$ représente l'hydrogène, un groupe alkyle inférieur contenant jusqu'à 4 atomes de carbone inclus, alcoxy inférieur contenant jusqu'à 4 atomes de carbone inclus ou un halogène de numéro atomique allant jusqu'à 53 inclus, $R_5$ représente l'hydrogène, un groupe alkyle inférieur contenant jusqu'à 4 atomes de carbone inclus, un halogène de numéro atomique allant jusqu'à 53 inclus ou un groupe trifluorométhyle, $R_6$ représente l'hydrogène, un groupe alkyle inférieur contenant jusqu'à 4 atomes de carbone inclus, alcoxy inférieur contenant jusqu'à 4 atomes de carbone inclus, cyano ou un halogène de numéro atomique allant jusqu'à 53 inclus, $R_7$ représente l'hydrogène, un groupe alkyle inférieur contenant jusqu'à 4 atomes de carbone inclus, un halogène de numéro atomique allant jusqu'à 53 inclus, un groupe (alcoxy inférieur)-carbonyle contenant jusqu'à 5 atomes de carbone inclus, carboxy, cyano ou alcanoyle inférieur contenant jusqu'à 7 atomes de carbone inclus et alk représente un groupe 1,3-(2-hydroxy)-propylène, ou leurs sels.

4. Procédé selon la revendication 1, caractérisé en ce que l'on prépare des composés de formule Ia dans laquelle $R_1$ représente un groupe alkyle inférieur contenant jusqu'à 4 atomes de carbone inclus, $R_3$ représente un groupe alkyle inférieur à chaîne droite contenant jusqu'à 4 atomes de carbone inclus, $R_2$ représente un groupe 5-tétrazolyle, $R_4$ représente l'hydrogène, $R_5$ représente un groupe alkyle inférieur contenant jusqu'à 4 atomes de carbone inclus, $R_6$ représente l'hydrogène, $R_7$ représente un halogène de numéro atomique allant de 17 à 53 inclus ou un groupe cyano et alk représente un groupe 1,3-(2-hydroxy)-propylène, ou leurs sels de bases.

5. Procédé selon la revendication 1, caractérisé en ce que l'on prépare le N-{3-[3-(4-acétyl-3-hydroxy-2-n-propyl-phénoxy)-2-hydroxy-propyloxy]-4-bromo-6-méthyl-phényl}-1H-tétrazole-5-carboxamide ou un sel de ce composé.

6. Procédé selon la revendication 1, caractérisé en ce que l'on prépare le N-{3-[3-(4-acétyl-3-hydroxy-2-n-propyl-phénoxy)-2-hydroxy-propyloxy]-2-cyanophényl}-1H-tétrazole-5-carboxamide ou un sel de ce composé.

7. Procédé selon la revendication 1, caractérisé en ce que l'on prépare le N-{3-[3-(4-acétyl-3-hydroxy-2-n-propyl-phénoxy)-2-hydroxy-propyloxy]-6-méthyl-phényl}-1H-tétrazole-5-carboxamide ou un sel de ce composé.

8. Procédé selon la revendication 1, caractérisé en ce que l'on prépare le N-{3-[3-(4-acétyl-3-hydroxy-2-n-propyl-phénoxy)-2-hydroxy-propyloxy]-4-chloro-6-méthyl-phényl}-1H-tétrazole-5-carboxamide ou un sel de ce composé.

9. Procédé selon la revendication 1, caractérisé en ce que l'on prépare le N-{3-[3-(4-acétyl-3-hydroxy-2-n-propyl-phénoxy)-2-hydroxy-propyloxy]-4-fluoro-6-méthyl-phényl}-1H-tétrazole-5-carboxamide ou un sel de ce composé.

10. Procédé selon la revendication 1, caractérisé en ce que l'on prépare le N-{3-[3-(4-acétyl-3-hydroxy-2-n-propyl-phénoxy)-2-hydroxy-propyloxy]-4-cyano-6-méthyl-phényl}-1H-tétrazole-5-carboxamide ou un sel de ce composé.

11. Procédé selon la revendication 1, caractérisé en ce que l'on prépare un composé pouvant être obtenu selon l'une des revendications 1 à 10 à l'état de sel de sodium, de potassium, de triéthanolammonium, de diéthylammonium, de diéthanolammonium ou de tris-(hydroxy)-méthylammonium.

12. Procédé de préparation de compositions pharmaceutiques, caractérisé en ce que l'on mélange un composé obtenu selon l'une des revendications 1 à 11 avec des produits auxiliaires et/ou véhicules usuels.